# EUROPEAN PATENT SPECIFICATION

(11) **EP 1 268 305 B1**
(45) Date of publication and mention of the grant of the patent: **12.09.2007**
(21) Application number: 01920648.1
(22) Date of filing: 21.03.2001
(51) Int. Cl.: B65D 81/00, A47G 21/18

(54) **INFUSION PACKET WITH USEFUL AND DECORATIVE ELEMENTS, SUPPORT MEMBER, DELIVERY SYSTEM AND METHOD**
AUFGUSSBEUTEL MIT DEKORATIVEN GEBRAUCHSMITTELN, TRÄGERELEMENTEN, ABGABEVORRICHTUNG UND VERFAHREN
PAQUET POUR SACHET A INFUSER PRESENTANT DES ELEMENTS DECORATIFS UTILES, ELEMENT DE SUPPORT, SYSTEME ET PROCEDE DE DISTRIBUTION

(30) Priority: 21.03.2000 US 192243 P
(43) Date of publication of application: 02.01.2003
(73) Proprietor: Stillman, Suzanne Jaffe, Los Angeles, California 90025 (US)
(72) Inventor: Stillman, Suzanne Jaffe, Los Angeles, California 90025 (US)
(74) Representative: Fiener, Josef
(86) International application number: PCT/US2001/009171
(87) International publication number: WO 2001/070591

(56) References cited:
- CH-A- 272 538
- DE-A- 4 001 500
- DE-A- 19 842 526
- DE-B- 1 045 314
- DE-U- 29 616 646
- FR-A- 2 671 332
- FR-A- 2 786 303
- US-A- 2 199 406
- US-A- 2 370 931
- US-A- 5 921 955

## Description

### Field of the Invention

The present invention relates to the an infusion packet as a delivery system for active ingredients such as pharmaceuticals, nutraceuticals, and/or dietary supplements for humans and animals.

An infusion packet according to the preamble of claim 1 is known from DE-A-198 42 526.

### Background of the invention

Infusion packets, water permeable coverings containing dried plant materials (e.g., a tea bag), have long been used to prepare infusions of various botanical origins. Tea is probably the most common constituent found in infusion packets.

Of historical note, tea is nearly 5,000 years old and was discovered, as legend has it, in 2737 bc. by the Chinese emperor, Shen Nong, who was both a skilled ruler and a creative scientist, required all drinking water to be boiled for hygienic precaution. One day as he stopped to rest and the servants began to boil water, dried leaves from a nearby bush fell into the boiling water. Brown color was extracted from the leaves and permeated the water. Even the curious scientist the emperor was interested and drank the resulting liquid. He found it remarkably refreshing and so tea was bom. In the 1600s tea became popular throughout Europe and the American colonies. In 1904, iced tea was created at the worlds fair in St. Louis.

Legend has it that as tea was an accidental discovery, so was the tea bag. Tea traders, who began selling tea in silk bags, invented the tea bag. Some customers dispensed with the step of opening the bags to obtain the tea within and simply dipped the entire bag into boiling water to prepare tea. Thomas Sullivan, a New York businessman, actually invented the modern tea bag in 1908. He started shipping samples of his teas in small silk bags for customer's approval, the noting that many customers actually preferred the samples because the tea bags were easier to use than loose tealeaves. The practice of putting tea into tea bags has given rise to one of the world's most creative packaging ideas.

The general notion of the tea bag is that an aliquot of herbal material is placed within a permeable package and dunked into a liquid-mostly commonly hot water. The hot water, and or a hot liquid, thus allows the various constituents to transfer more easily from the permeable package into the liquid. This is in addition to the egg-shaped, perforated, usually metal containers for tea leaves to be submerged into cups and/or tea pots for the preparation of tea. Further there exists filter inserts (funnel-shaped members) which are clamped into a holder and then receive tea leaves.

Tea breaks down into three basic types: black, green, and oolong. In the U.S., over 90% of the tea consumed is black tea, which has been fully oxidized or fermented. Greed tea skips the oxidizing step. Green tea is a staple in the Orient, is gaining popularity in the U.S. and other parts of the world due in part to recent scientific studies linking green tea drinking with reduced cancer risks. Oolong tea, popular in China, is partly oxidized.

While flavored teas evolve from these three basic teas, herbal teas contain no true tea leaves. Herbal and medicinal teas are created from the flowers, berries, peels, seeds, leaves, and roots of many different plants. The herbal materials placed within an infusion packet are usually selected to impart active ingredients, interesting color, aroma, and flavor to the infused liquid. To this end flavorful or fragrant materials such as citrus peels, flowers, and or herbal materials such as mint, and jasmine are frequently used. However, infusion packets are also known to provide sources of "functional" or medically active ingredients as exemplified by a leading herbal tea company, Traditional Medicinals, a company which produces over thirty different herbal teas to meet a wide variety of health needs. Their product line includes Think-02 with ginkgo biloba, an herb proven safe for mental alertness and memory; Echinaacea Plus tea for when a cold or the flu starts; Throat Coat contains Pau d'Arco to coat the throat and a double bag of American Ginzing for an extra big kick.

From the first used tea bag going forward the tea bag has undergone many improvements both in materials to exotic crimping methods for closing the seams without adhesive or sewing. Literature is replete with various methods, designs, fabrications etc. designated to address better infusion. U.S. Pat. No. 5,674,544 to Shakspeare, (Tidy Tea Limited GB) discloses a compressible infusion package using a drawstring method to address better infusion. Another drawstring method is reflected in U.S. Pat. No. 5,951,452 to Stevenson, (Tetley GB Limited). Another to 5,989,602 to Drury and Dante, (Lipton) uses a drawstring whereby allowing the tag card to be detached to draw out further portions of the drawstring to contract the packet and squeeze out excess moisture after infusion.

It has been noted that basically commercially sold tea bags or infusion packets are relatively the same shape while sizes may vary. The usual shape is square or slightly rectangle. Recently some round shapes are on the market looking like a filled pancake. U.S. Pat. No. to 5,910,247 to Outterside reflects a two element filter bag which includes a cylindrical outer element and a generally tubular inner element. Other shapes of noteworthlness am in the flow through category. These are functional having been designed for better infusion and no relation to creative appeal. U.S. Pat. No. 5366,741 to Van Der Zon (Thomas J. Lipton Co. NJ) teaches a V-fold thus creating a duel compartment tea bag. Another U.S. Pat No. 5,852,917 to Romagnoli (I.M.A. Industria Machine Automatiche S.p.A. (Bologna IT) titled Method of manufacturing double-chambered infusion bags by folding.

Later came the advent of different materials for the construction of the tea bag. U.S. Pat. No. 5,601,716 to Helnrich and Roland, (Papcel of Gemsbach, DE) discloses a filter material a composed of both natural and synthetic plastic fibers.

### Summary of the Invention

It is the object of the invention to provide infusion packets individually and or in combination pharmaceuticals, nutraceuticals, herbals, pro-biotics, prebiotics, amino acids, digestive enzymes, soluble fiber and/or any dietary supplements by infusing the aforementioned into a liquid. In particular it is a system intended to impart feelings of well being, improve health, and quality of life by delivering into liquid (mainly water) both desired and necessary ingredients.

Further, the present invention, as defined in claim 1 and 5, respectively, may accomplish the aforesaid with one infusion packet and/or more than one packet thus comprising a daily, weekly, or monthly supply. Each good tasting therapeutic infusion packet is useful for administrating active ingredients especially to those who refuse to swallow tablets or may have difficulty doing so, or for those who simply will not consume carriers for active ingredients unless they have pleasing taste characteristics. Ultimately, better taste translates to a greater likelihood of individual consumption, thus a greater realization of the health benefits. Ergo a high degree of consumer compliance. Further in its entirety, and/or in combination with more than one infusion packet, and/or with the packaging container may bring forth one or more of the following elements, color, texture, advertising, promotions, games, interactive entertainment, and/or "edutainment".

The inventor is most concerned here that the use is mainly to provide a functional beverage suitable for drinking which beverage contains metered amounts of active ingredients for targeting specific health problems or for providing general with healthy benefits and/or enjoyment. The current invention approaches its goals through several different modifications of the traditional Infusion packet.

The invention is concerned with contents of the infusion packet whereby specialized health enhancing ingredients-in particular soluble fiber-are added to the infusion packet. The reader's attention is directed to PCT/US01/05630 with a filing date of 22 Feb 2001 for more details on the use of soluble fiber.

Similarly, the infusion packet itself can be incorporated into a toy or some other attractive object. In this situation the packet can carry printed indicia to amplify this point. If an impermeable material is used, the necessary perforations can be arranged in a pattern to enhance the overall design or even to provide "special effects" (such as color diffusing from a perforation when the packet is placed in liquid.)The packets can be similarly compartmentalized to control the interaction of the active ingredients contained within.

A new component, here called a support member, can be used to enhance the overall functionality and attractiveness of the inventive packet(s). A support member may be a tool that holds the infusion packet for proper placement in liquid. The support member may also be used to combine one or more separate packets into a functional unit.

One or more infusion packets with or without the packaging container may or may not include an additional support member which is intended to support the ease of use and, independently or simultaneously, include one or more decorative, functional, entertainment, and or educational elements.

Within a container holding the infusion packets, with or without a support member, there may be extra items necessary to execute an extracurricular objective such as a game.

The packet itself may be specially sized and shaped for insertion into a water bottle or similar device. In such a case the tag can be adhesive backed for attachment to the outside of the bottle. The packet for bottle use can be designed to change shape in liquid so as to be unable to pass back through the bottle's neck and into a drinker's mouth.

The packet may be equipped with one or more separate compartments for the single-unit dose per compartment regardless of form. The active and accessory ingredients (color, aroma etc.) may be mixed or separated in different compartment as desired and/or required. The components may be in dried form (powder, beads, granules), liquid form, gel form, tablet form, capsule form, or any form, that may or may not have effervesce. The user may have the option of determining how much of the active ingredients are preferred through the transportation process from inside the packet to the infusing liquid by how long the packet is left within or in contact with the liquid.

Certainly it is easily understood as to why there is a filtering mechanism when herbals and or tea like constituents are used. It may become less apparent when ready to mix ingredients are supplied in powder, granule, crystal, etc form. All one would have to do is tear open the packet and pour the continents into liquid. While this may be an effective method in some instances, it does not come without serious drawbacks. Such drawbacks are that the contents might spill and create a messy situation. Additionally some of the contents may remain stuck in the packet and therefore not available to the liquid.

Opening a packet whereby you have only one compartment does not allow for the separation of ingredients until mixing. This can be most critical in many formulations. Further, ingredients may have a longer shelf life when contained in an infusion packet in separate compartments. Further, having a portion of a specific amount of material enclosed within an individual compartment of an infusion packet further ensures a potentially more sanitary delivery into the liquid. This is especially valuable for children who use unclean hands to touch many foods, seniors, and those who have manipulation problems as related to hand dexterity.

It is of critical importance to the invention that every effort is made to provide consistency in ingredients within each packet. Recognizing that there is a possibility of the consumer not receiving enough of an ingredient in the course of a day by other means and whereby the infusion packets contains needed supplements it is equally significant to be sure that there is not a negative response to receiving to much of one or more ingredients.

Additionally, if one wishes to look at a daily regime of proper hydration along with supplementation it is conceived by the inventor that a daily pack of infusion packets be packaged together. This can be broken down by time of day into two or more synergistically designed formulations. It is easy to understand that a consumer may have 4-6 or more infusion packets in a daily pack. Each can be a separately designed supplement group and travel with the consumer far better than ready mixed bottled drinks where by active ingredients are in solution with sweeteners, food acids, chemicals, etc.

To facilitate better use and/or for fun and enjoyment it is conceived that effervescence be used. Effervescent ingredients (powders, granules and/or tablets) are very hygroscopic and to be sure that the integrity of the effervescence would be maintained a special outside wrapper would have to be used. Then and only then could effervescent powder, granules, and or a tablet be considered.

Further it is conceived that the invention include encapsulations which may be uniformly released and or timed released. The uniformly released may be nothing more than bursts of flavoring where as the time release may provide the ability to suspend the release of active ingredients until they reach the gut. This is most valuable in the delivery of some pre-biotic and or pro-biotic formulas. The encapsulations may be imbedded in the filtering (envelope) material and/or contained within the mixture inside of the porous packet. Upon contact with the liquid the encapsulations may be released into the liquid from the envelope layer and or travel through the pores into the liquid in whole. It is further contemplated that upon contact with the liquid the encapsulations open and only the ingredients contained within travel through to the liquid.

Further, the encapsulations may be used to preserve and or better deliver the active ingredients. These encapsulations may become active when the packet comes in contact with the liquid.

In addition, flavoring, coloring or nutritionally active components can advantageously be microencapsulated to ensure stability. The microcapsules can be formulated to release their contents when liquid is added (either by fracturing or dissolving). Alternatively, the microcapsules can be formulated within the micro-beads, or other components to be released intact upon the addition of liquid. In that case the microencapsulated ingredients readily pass through the packet's covering and are ingested with the liquid. The microcapsules then release their contents (up to several hours later) at predetermined points along the digestive tract. In this way it is possible to precisely deliver a labile component.

Various encapsulated ingredients can be included. Encapsulation controls stability and compatibility of ingredients as well as timing ingredient release. Further if a double membrane is used then the encapsulations may be of colored, varied and/or consistent, "beads" locked between all or part of the walls of the membrane which may or may not be fully transparent. The inside of the packet still contains all that is mentioned. These encapsulations may follow all of the claims here for encapsulations.(shells may stay most likely, but make if cover everything) adding further actives to enjoyment.

The inventor's intent is to provide an incentive towards meeting daily hydration requirements. This is especially critical for children, seniors, and the health challenged as well as those individuals who do not like water and/or have not realized the importance of drinking water or hydrating beverages on a regular basis.

Active ingredients as well as aromas may be impregnated into the infusion packet to be infused into the liquid. Aromas may not be used to be infused into the liquid as they may impart an undesirable taste. It is then conceived that other components of the infusion packet and or supporting member which does not come in contact with the liquid be impregnated with aroma "aromatherapy" and emanate from the immediate vicinity of the liquid.

Alternatively, the design and/or color may dissolve off the packet in whole or in part into the liquid and go undetected in the liquid. This can be most fun for children as they may see their favorite character slip into their drink. Not unlike polished stones we see today in the stores which have inspirational words, like the word *"believe"* carved into them and/or messages such as *"an ounce of prevention is worth a pound of cure"* on wooden plaques these same words/messages may wash into the beverage. Additionally in the reverse when the infusion materials in contact with the liquid secret messages can be revealed

For the health challenged, especially children, who are in need of a naso-gastric feeding tube on a regular or part time basis often balk when the tube is to be put into place. Possibly if they could be a part of the design of the liquid to go into their tube, either pushed in or accommodating a gravity flow of a just prepared mixture, they may be more responsive. Certainly a little entertainment, pre-passing of the tube, might relax the nervousness of the soon to be performed procedure. Especially important when the need to be relaxed is critical to success. The same holds true for those on gastro-enteral feeding tubes. This way the patient may be part of the solution as compared to part of the problem.

An object of the invention is to provide ingredients, which are dissolvable within the packet as well. This would mean that by the nature of physics the ingredients would attract, draw, the liquid into the packet in a greater than normal proportion. Then when you lift the packet the liquid spills out most likely through designated pores which are sized accordingly.

The infusion packets may be packaged within a separate wrapper of any material appropriate to the contents. If so wrapped the wrapper may contain promotional material and or any of the characteristics that are on the packet and or the tab (fob) at the end of the string. The packaging of the infusion packets in a separate wrapper or stacked in boxes of a predetermined amount is obvious to those skilled in the art of tea packaging.

Entirely new packaging methods may be designed that are both ingredient and intent specific. Each infusion packet contains one or more active ingredients not normally packaged together or hitherto activated by this method. All ingredients may be delivered dry or in any form that will allow the transfer from inside the packet to the liquid to occur as dictated by the specifics of activity and/or desired effects immediately or a predetermined, or random determined length of time.

Further, at the other extreme many of our seniors begin to act like children. As one ages the thirst mechanism decreases according to Dr. John Greenleaf NASA. The reason for this change is not known.

Sugar is not on the "good for you" list in our society today yet many of the older population does not understand this. Many popular beverages avidly consumed by seniors are high in sugar. Additionally these beverages contain ingredients, which may not mix with the many medications taken by most seniors. Moreover, such beverages are very expensive, and our older population does not have discretionary income. So the seniors are left with what the children have plus the disadvantages, which go with liquor, coffee, and even tea with a huge amount of caffeine.

Thus it is the object of the invention to provide a unique delivery system for all humans and animals but also to look towards the senior's marketplace by the design of the beverage, the artwork etc. Many seniors are afraid of hot liquids because of infirm hands and failing eyesight yet they delight in custom and would so enjoy a vehicle they recognize and can use most frequently.

### Brief Description of the Drawings

Figure 1 shows a perspective view of an infusion packed designed to be used in a narrow necked bottle. Fig. 1A shows the device with a "sports top". Fig. 1 B shows a regular bottle top. Fig. 1C shows the infusion packet in a swelled confirmation following contact with iiquid-this configuration is unable to pass accidentally into the user's mouth or plug the sports top.
Figure 2 shows a sectional view of the device of Fig. 1. Fig. 2A is taken from Fig. 1A and Fig. 2B is taken from Fig. 1C. Fig. 2C shows a detail of color streaming from a figure shaped infusion packet. Fig. 2D shows the infusion packet after all of the color has left the packet to color the surrounding liquid.
Figure 3 shows a perspective view of a support member shaped as a toy figure. This support member is attachable to a drinking straw and also alternatively holds or contains the infusion packet.
Figure 4 is a sectional view of the support member of Fig. 3 to show one placement of the infusion packet.
Figure 5 shows an alternative design for a toy-like support member.
Figure 6 shows an infusion packet with a hidden design printed on the packet.
Figure 7 shows the packet of Fig. 6 after immersion in liquid whereby the hidden design is now visible.
Figure 8 shows an infusion packet with a visible printed design which is related to the tag which is a charm bracelet charm. A clip tag is also pictured.
Figure 9 shows a support member which encloses an infusion packet.
Figure 10 illustrates a charm bracelet composed of charms which serve as infusion packet tags (as in Fig. 8).
Figure 11 illustrates an infusion packet according to the present invention together with a support member which is used to connect diverse infusion packets. Fig. 11A shows a surface view, and Fig. 11 B shows a sectional view to expose the diverse contents of the infusion packets.
Figure 12 illustrates an infusion packet wherein the packet is formed as a figure and the tag is a counterweight.
Figure 13 illustrates a multiple package of infusion packets which form a number game wherein individual packets reveal numbers related to their tags upon immersion.
Figure 14 illustrates another game wherein tags serve as cards with the winner finding a "preferential card".

### Detailed Description of the Invention

For interpretation and clarity the inventor has chosen to use the word "infusion packet" to included other common terminology such as tea bags and or sachets. The inventor feels that the term, "tea bag" may prejudice the reader towards a belief that only an aliquot of herbal materials are enclosed within and, perhaps more specifically, used for drinking. While the term "sachet" also refers to a small bag it is most commonly used when referring to a small bag containing a fragrance and whereby the sachet device is used to dispense fragrance.

For clarity the inventor will refer to beverages as to include anything, regardless of a food qualification category that will be brewed and or dissolvable on total or in part and pass through a permeable membrane or mesh into liquid. Encompassed herein would be a category commonly known as soups and broths.

For clarity the inventor has chosen support member to mean any device regardless of the material that is used in conjunction with an individual infusion packet and or group of packets to support the packet or packets. For example, as a tool to handle the packets or to join them together. Most commonly a support member will have toy-like aspects.

The present invention is targeted at humans of all ages, and even to animals because many pharmaceuticals, nutraceuticals, and or dietary active additives only demonstrate activity, and or show better stability when administered in a freshly dissolved state. The present invention is geared towards preserving the integrity, activity, of the ingredient(s) both in terms of how the individual packets are packaged and, additionally, how the ingredients within the packets are stabilized until the packets are used.

The primary interest of the inventor is to provide a safe, easy, efficient product which is convenient and fun to use while at the same time providing opportunities for beverage development with more natural and healthful ingredients and reduced sugar loads to target specific needs and/or ages of consumers. Ensuring hydration is a very important part of the inventive goal.

A portion of the invention is directed primarily to an adult product that mainly uses hot liquids (mainly water) for activation (extraction or dissolution of the active components). However it is noted that this adult population are now consuming a tremendous amount of ready to drink teas, coffees, sodas, new age beverages, and energy drinks sold today. Some of these claim one or more health benefits and are labeled as such but with those purported benefits comes a serious amount of sugar and also often caffeine, a stimulant which is also dehydrating.

### Bottled Water and Water Bottles

With many consumers today drinking bottled water and with the inventor being prejudiced towards hydration, and with the fact that hot water is not necessary to reconstitute many formulations it is therefore ideal to have a shaped infusion packet that will readily slip into a bottle. While it is possible to "stuff' an existing infusion packet, "tea bag", into a bottle one risks the chance of tearing etc. thus rendering it unusable. Further, all of the infusion packets that exist today basically need a very warm, even better a boiling hot, solution to activate. The polyethylene terephthalate (PET) bottles cannot be heated as the material will not allow such a procedure, nor can water over 160 degrees F (70 degrees C) be put in them successfully. While it is possible to put one of these bags into a bottle and let it sit for a length of time, this process has been called making "sun tea" from a time when individuals would put tea bags in water and then let them brew in the sun, the inventor discovered that consumers do not want to bother setting up this process way in advance, over overnight, much less time in the sun.

The invention set forth here easily accommodates the opening of a conventional PET bottle. The size of bottles are determined by the volume of liquid they hold. Most common today are bottles that hold 16.9 oz. (0.5 l), 20 oz. (0.61), 23.5 oz. (0.7 1), and 50.7 oz. (1.5 l). of water. It is of note that they all have a standard opening which will also accommodate the very popular sports cap. Depending on the volume of liquid, in relation to the nature of the contents, the weight of the product will be in the range of 0.1 grams to 50 grams. The infusion packet may or may not have a string which may or may not have a tag, or tag-like arrangement at the end. While this invention is specifically designed to go into PET bottles of water it may further go into glass bottles of water and/or plastic/glass bottles of any existing drinks to add nutritive, decorative, flavor and/or aromatic stimulants should they be so desired.

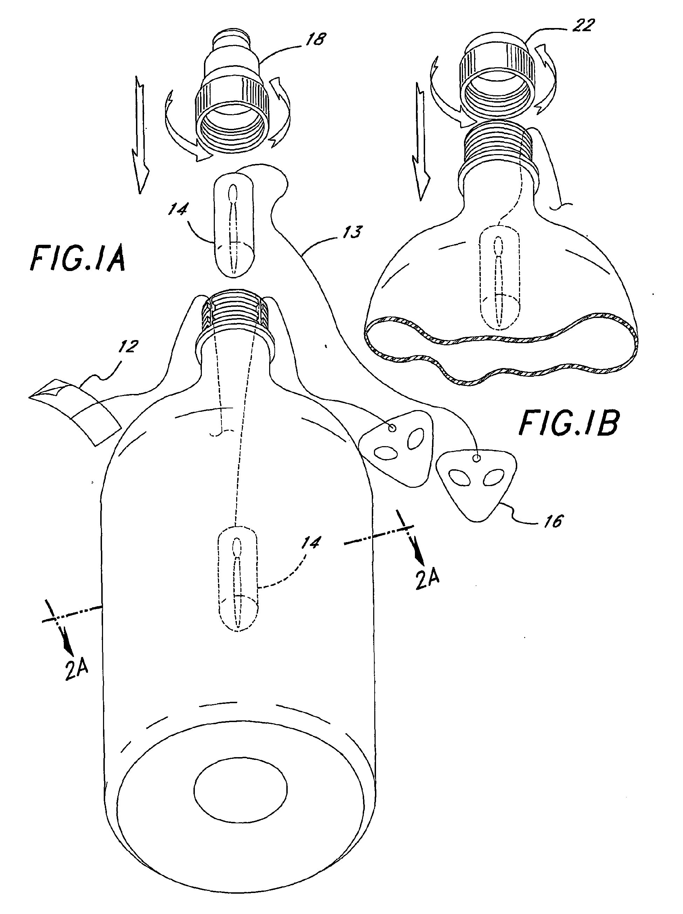

The basic structure of a bottle infusion packet 14 is shown in Fig 1A in place in a PET water bottle with a "sports top" 18. (Fig 1 B shows a regular top 22). The packet 14 is sized and shaped to fit through the neck of the bottle. A tether 13 (usually a thread or string but a chain, monofilament or other connector could be used) connects a tag 16 with the packet 14. Here the tag 16 is a decorative (possibly collectable) "alien face". Fig. 1A shows the packet 14 slipping through the neck of a bottle. The tag may also be adhesive backed as is tag 12 to ensure fixation on the bottle side. It is also noted that there is a concern as to when the drinker drinks the liquid if he has not removed the packet swallowing can be a danger. An edible, dissolvable film containing the actives is one way to address this situation so that the packet would dissolve completely leaving no residue. Using a sports cap 18 to limit the opening of liquid going into the mouth is another potential solution. A system with enhanced safety would include, as an example, a compressed sponge or sponge like material attached to, or inside of the infusion packet, which would absorb liquid and render the item too big to go back through the opening of the bottle.

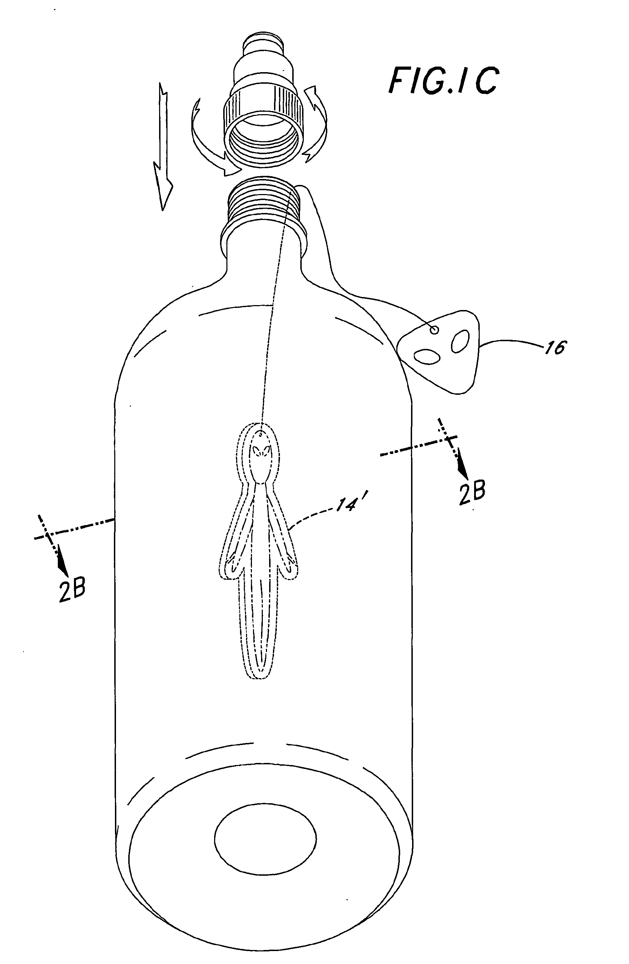

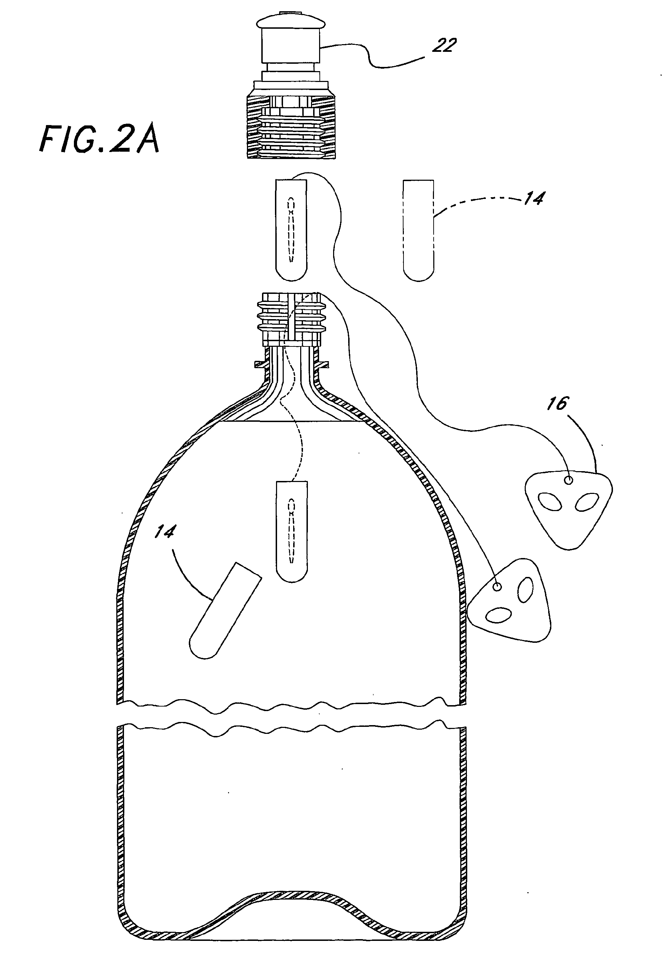

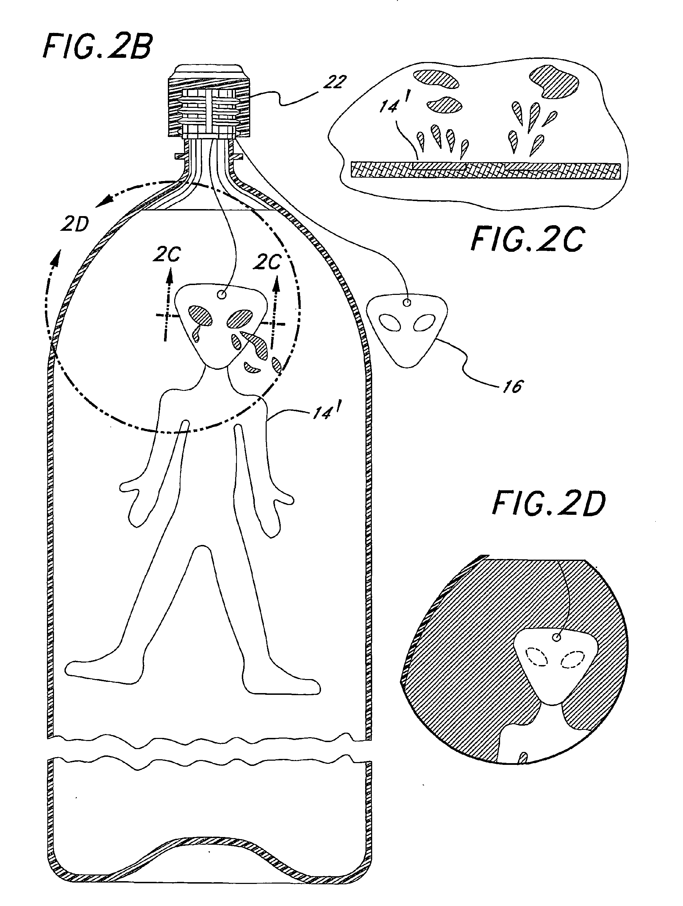

It is further conceived that a sponge, and/or a sponge like material, be impregnated with encapsulated active ingredients whereby both are delivered into the bottle at the same time. The sponge would expand and release the actives into the bottle. This is illustrated in Figs. 1C and 2B where the packet 14' has expanded to preclude reverse passage through the bottle neck. Note that expansion has revealed a specific design to the packet 14 which now represents an "alien" in keeping with the alien face tag 16. Figs. 2B-D show an additional aspect of the invention wherein the packet forming the alien head contains perforations through which color (alien tears) is emitted when the packet imbibes fluid. Fig. 2D shows all the color now in the surrounding solution.

It is possible to use the infusion packet as related to a drinking bottle in the following manner. The bottles can be made available with the addition of the infusion packet already inserted in the bottle, ready for activation, whereby the consumer can add water or any other hydrating liquid to activate (e.g., release the contents of the packet into the solution). The bottle can be filled to any level however the suggested level would be pre-marked on the bottle. If an infusion packet is used which contains an effervescent composition it will be most important not to overfill due to the fact that the effervescence will take up headspace.

While it is possible to present this invention with the infusion packet inside the bottle ready to accept the liquid it may not be practical and or safe to do so. Therefore the following is a variation on the same invention. The infusion packet may be provided with the bottle but not inside of it. Packaging technologies and blister pack technology is so advanced whereby this becomes economically practical. Additional advertising, coupons, promotions, or the like can also be added at the same time.

Additionally, as a consumer item, the packets may be made and sold individually or in sets either mixed and/or of the same blend. This would allow the consumer to efficiently buy the drink mix of choice, and purchase a bottle of water or fill an existing bottle of water say from a home delivered 5 gallon (20 I) jug. Suggested mixing instructions, including but not limited to a volume of liquid to be added would be printed on the packet itself and/or accompanying literature. Along with these packets could be included advertising materials, coupons, samples of additional products by the company, promotional material, etc. In a retail venue it would be most appropriate to have these made available in close proximity to where bottled water is sold, and or at best by the cash register.

### Children

Of keen interest to the inventor is the development of products specifically geared towards the younger generation as well products geared towards aging seniors. It is believed by the inventor that these groups need special dietary support.

Children certainly like parties, celebrations, and ceremonies, especially those revolving around known holidays but also need good quality beverages everyday especially for ensuring optimal hydration. Children only have beverage options within the scope of what is commonly available: milk, mostly cow, some goat, soy, rice, etc., soft drinks, which, are known to be sugar laden and often also loaded with caffeine, fruit juices, fruit juice blends, juice drinks (water with a small percentage of fruit juice mixed in), and drinks (generally flavored sugar).

An entire segment of a recent television show, 20/20, featured data showing how bad caffeine is for children, and in fact the popular soft drink, caffeinated Mountain Dew^{®}, cannot be sold in Canada. Fruit juices while containing some vitamins are very high in sugar. The popular fruit drink Hawaiian Punch^{®} and Kool Aid^{®} drink mixes are basically, little more than sugar and flavoring. Hyper-active children and children with attention deficit disorders are negatively impacted by excess sugar. While some beverages, such as sodas, with artificial sweeteners contain virtually no calories they do contain potentially hazardous chemicals for sweetening and many also contain caffeine.

Many of the beverages provided for children are high in calories. Many of the beverages fill children with the "empty calories" so that they do not have room for healthy foods.

"Liquid Candy" is the name that the United States Government has used to refer to these sugar loaded non nutritional sodas still popular today. In a recent article Liquid Candy How soft drinks are Harming American's Health by Dr. Michael E. Jacobson of the Center for Science in the Public Interest (CSPI) states that, "carbonated soft drinks are the single biggest source of refined sugar in the American Diet". More alarming is the fact that soft drinks are the fifth largest source of calories in adults. Further alarming is the fact that children start drinking soda pop at a remarkably early age.

The problems associated with these sugar laden soft drinks and the increase of empty calories found in these sodas are as follows: obesity, decreased bone health, tooth decay, heart disease, Kidney stones. Dena Mehlberg RD of the Froedtert Memorial Lutheran Hospital in Wisconsin advises consumers to get enough fluids each day (the average person needs 8-10 cups a day) and make healthier choices when selecting a beverage.

The inventor here hopes to dissuade, especially the younger population, from "Liquid Candy" and in some instances fruit juices which have a high sugar content and none of the fiber benefits as compared to eating the fresh fruit itself. While milk is an important source of calcium for those who are lactose intolerant the lactose in milk can present digestive problems in Asians, Hispanics, and Blacks.

Further, many of these beverages are not hydrating because they contain excess salts, sugars or diuretics (e.g., caffeine).

The inventor is extremely concerned that there exist the proper nutrition for children, especially health challenged children and those of indigent and/or low income families.

Additionally that children are hydrated properly. For an active child weighing 50 pounds one quart of water is needed per day. Water is not the choice of children. Children basically drink water from drinking fountains at school, the park, etc. They especially need hydrating beverages when they are sick and especially when they have high fevers. It is most difficult to get children to drink water at all let alone at times of illness. Parents and/or caregivers usually give water with vitamins and necessary medications, but just like the drinking fountain situation, children usually consume this water in inadequate sips.

There are many powdered beverages in the marketplace that must be reconstituted prior to consumption. Some of these are meal replacement or diet powders such as Slim-Fast^{®}. Recently several similar products have come to market for the younger set. These are more of the meal replacement. Kids Plex^{®} by Naturade^{®} and Animal Parade^{®} by Natures Way^{®}.

In the past to encourage children to do things they aren't normally inclined to do parents and/or caregivers have contrived all sorts of games and offered rewards. A drop of candy was place at the bottom of a cup, often in the case of milk, as a reward for drinking the entire beverage. The present invention includes a beverage delivery system that, while it is designed for all humans and animals, is particularly appropriate to the children's marketplace.

Children like to feel grown up and love to imitate well-known grown up activities. By presenting a new and novel way for them to make, reconstitute and/or brew their own drinks we are providing a grown up activity with good intentions. We are an interactive society and this is a personal interactive experience, which can be done alone or shared with others. For children especially it is most valuable to have an infusion packet that will allow the process to occur in other than potentially burning hot water. Burns are always a concern of parents. Besides hot liquids are usually sipped and children, unless pretending such as at a tea party, usually don't sip.

### Hydration and Soluble Fiber

The inventor is concerned about hydration as related not just to the hydration of the younger population but the entire population now especially towards an aging population. It seems that as we age we loose our thirst mechanism but not our need for hydration.

Water is the media for biochemical reactions, is required for expiration, moistens oxygen for breathing, protects and cushions vital organs, helps the body absorb nutrients, removes wastes, cushions joints, carries oxygen and nutrients to all cells in the body and provides an efficient cooling effect. Further the human body contains between 55-75% water depending on the ratio of body fat to muscle. The human brain is 75% water, blood 92% water, muscles are 75% water, bones 22% water. When dehydration occurs the following symptoms may present: vagueness, discomfort, increased pulse, nausea, dizziness, headache, labored breathing, walking difficulties, twitching, and ultimately when ½ to 2 liters of fluid are lost it can be life threatening. When organs are 5% dehydrated, negative changes begin to occur. A 2% reduction in hydration equals a 7% reduction in endurance. Dr. John Greenleaf, NASA, in U.S. Pat. No. 5,447,730 and authored literature addresses the importance of hydration. Dr. Greenleaf further notes that when organs are 5% dehydrated negative reactions begin to occur. The readers attention is drawn to "Problem: thirst, drinking, behavior, and involuntary dehydration " by John E. Greenleaf (Medicine and Science in Sports and Exercise, 24:645 (1992).

Of further significance to the inventor is dietary fiber, mainly soluble fiber which has been stressed by several health organizations of the Federal Government. The Dietary Guidelines for Americans published jointly by the U.S. Government Department of Agriculture and Health and Human Services, and the National Cancer Institute recommend that all American's increase these daily intake of fiber. Health care professionals conclude that the American diet provides only about half of the suggested daily requirement of 20-35 grams. Soluble fiber is now known to address prostate and colon cancer, stabilize blood sugar, curb appetite, prevent weight gain by slowing the rate of digestion (transit time of food through the colon), prevent obesity, lower blood cholesterol, lower risk of heart disease, expedite the removal of potentially dangerous toxins (e.g., carcinogens and heavy metals), bind to bile salts and help decrease the risk of gallbladder disease, and promote regularity.

Of specific importance to diabetics high fiber improves glycemic control, as evidences by decreases in the mean daily postprandial rise in glucose. (The New England Journal of Medicine, May 11, 2000, p.1392-1398.) Conclusions: A high intake of dietary fiber, particularly of the soluble type, above the level recommended by the American Diabetic Association, improves glycemic control, decreases hyperinsulinemia, and lowers plasma lipid concentrations in patients with type 2 diabetes.

Soluble fiber comes from a wide range of plant sources. Water-soluble plant pectins and pectic materials, galactomannans, arabanogalactans and water-soluble hemicelulose can act as soluble fiber. Many plant "mucilages," gums, and soluble polysaccharides found in grains, seeds, or stems such as psyllium, guar, oat (beta glucans), astragalus (gum traganth), gum ghatti, gum karaya (Sterculia gum), and gum acacia are also soluble fiber. Algal polysaccharides such as agar or carrageenan also behave as soluble fiber as do other indigestible carbohydrates, such as maltodextrins or dextrins, produced by chemical or enzymatic digestion (e.g., partial hydrolysis) of starch, gums and other carbohydrate polymers. Soluble cellulosic ethers and other derivatives such as carboxymethyl cellulose behave as soluble fiber as do indigestible carbohydrate polymers artificially prepared using bacterial enzymes. Non-digestible storage carbohydrates such as inulin are also important soluble fibers. A number of companies are now providing an entire range of "soluble fiber" materials. For example, TIC Gums of Belcamp, Maryland, Novartis Nutrition of Minneapolis, Minnesota and Imperial Sensus of Sugar Land, Texas provide soluble fiber compounds of food grade.

Soluble "fiber" is known to provide a novel opportunity for improving the characteristics of fiber-poor refined foods. Fiber was removed from food products because in many cases it made the foods coarse, unpalatable or difficult to process. Adding insoluble bran or other similar fiber to foods may provide more roughage but can also degrade the favorable properties of the foods. For example, cakes or pastries made from flours high in insoluble fiber may have inferior taste and texture. Excess insoluble fiber may upset the digestion and lead to a number of digestive problems. On the other hand, soluble fiber is generally well tolerated, often improves the texture or other physical characteristics of the food product and is generally innocuous. Consequently, there are a growing number of food products, ranging from baked goods to "shake-like" beverages, contain added fiber in the form of soluble fiber. Soluble fiber can restore the benefits of fiber to our highly refined diet.

The present inventor has realized that a properly formulated infusion packet is the ideal vehicle for administering nutritional supplements such as soluble fiber. Each packet represents a precisely measured delivery device for nutritional supplements. To the inventor's knowledge this vehicle has not been taken advantage of hitherto. By adding various water-soluble functional nutritional supplements to an infusion packet, it is possible to provide a unique delivery system for the supplements. Soluble fiber is just one case in point. It is well known that dietary fiber is absolutely essential for optimal health. Dietary fiber supports regularity, which minimizes the absorption of toxins. Dietary fiber is known to retard the absorption of lipids and lower serum cholesterol. Dietary fiber is known to moderate the rate of carbohydrate absorption from the digestive tract and thereby ameliorate swings in blood sugar. It has been demonstrated that besides the already listed benefits of fiber arabanogalactans also possess the power of stimulating the immune system. These benefits are well known for humans but are also available to animals that have digestive systems substantially similar to humans (e.g., dogs, cats and some other domestic animals). Therefore, the current invention is useful for these animals as well as for humans.

Surprisingly, water-soluble carbohydrates can act as dietary fiber and provide the beneficial effects just listed. The problem has been to provide a convenient vehicle for soluble fiber. An infusion packet provides the ideal vehicle for soluble fiber. A measured dose of soluble fiber can be enclosed in a packet. The precise dose per packet is a matter of design choice, but dosages of between 0.1 and 50 grams are readily accommodated and function well with either hot or cold liquids (depending on the exact fiber chosen). Other, more traditional, infusion materials can also be included. For example, it is relatively simple to produce a packet that provides "fiber tea." By adding hot water to the packet one rapidly produces a tea beverage with the added benefits of soluble fiber. The tealeaves and the soluble fiber (depending on the fiber type) may be mixed together within the infusion packet. Alternatively, the packet can have a plurality of compartments so that the tea (or other ingredients) can be kept separate from the soluble fiber until the actual moment of preparation. It is possible to use several different types of soluble fiber and keep them separate in individual compartments. If any of the soluble fiber compounds used are hygroscopic, the entire packet can be sealed in a humidity-proof pouch.

Although traditional uses of infusion packets require hot or warm water, certain soluble fiber carbohydrate materials are very soluble in cold water as well. Thus, it is possible to provide a soluble fiber infusion packet that can be used in cold water. Additional water-soluble flavoring agents and/or sweetening agents can be included to provide a tasty fiber beverage that is produced by dropping the infusion packet into cold water. Again, the various components may be mixed together in the packet or can be compartmentalized. Regardless of water temperature some of the soluble ingredients inside the infusion packet can advantageously be provided in the form of tablets, pellets granules or beads as opposed to powdered ingredients. This mitigates against the ingredients sifting through the pores of the covering material. As is known in the art, certain micro-bead configurations can be formulated with extremely rapid solubility properties.

Therefore by adding soluble fiber to the aliquot of constituents used in the infusion packet we are able to address the health benefits of fiber along with the properties of the fiber selected. Depending on the fiber(s) used it may not be appropriate to place them (one or more combined) directly in contact with the other ingredients. It is to this that the inventor looks at compartmentalizing as much as possible to protect each ingredient. Not just the ingredients used but the manner as to how they are delivered is critical. Depending on the fiber used it may carry with it other unique characteristics, such as serving as a pre-biotic, pro-biotic, an immune enhancer, and may also contribute to enhanced mouth-feel.

The carbohydrate inulin, which occurs in over 36,000 plants is all natural and non-digestible by the human digestive system. It has been consumed for centuries by entire populations as a main staple in various food sources, such as onions, wheat, J. artichokes, asparagus and others.

However current consumption from natural sources is not large enough to provide efficacious inulin dose (approximately 5 g/day minimum for improved physiological health as a unique soluble dietary fiber and preferred food for healthy intestinal bacteria. (bifidobacteria and lactobacilli).

In addition inulin provides a myriad of health properties for which under DSHEA and further clarified by the FDA 4/29/98 in 21 CFR Part 101 Food Labeling: Nutrient Content Claims, Definition of Term: Healthy, statements of structure or function may be made for mainstream inulin-containing products, e.g. "promotes growth of beneficial bacteria such as bifidobacteria", "bifidogenic", "helps to maintain a normal, well balanced gut microflora", helps maintain intestinal flora", "stimulates natural Bifidus flora", "inulin is efficiently converted to short chain fatty acid, "helps maintain cardiovascular function and a healthy circulatory system, "helps promote urinary tract health", "helps maintain a healthy cholesterol level", helps to regulate blood glucose level", "helps maintain regularity, "helps improve mineral bioavailabillity", "supports the immune system", are allowed.

Further impressive literature is replete with the benefits of inulin hailing its ability to suppress pathogenic gut microorganisms and their toxins, prevent ulcerative colitis and malabsorption, demonstrates positive influences on blood sugar regulation and also balance insulin for diabetics. Further to improved calcium absorption for osteoporosis and immune activation as related to disease prevention, anti-tumor effects, reduction in food allergies, and potential help for autoimmune diseases like Crohn's and rheumatoid arthritis.

In recent years scientific evidence for reducing serum lipid levels in man and animals using inulin has grown. (Shown to lower LDL and raise HDL)

In food and/or beverage it has neutral taste, odor, and color and is ideal to be incorporated into an infusion packet blend.

The inventor has noted the following patents related to inulin as relevant; U.S. Pat. No. 5,972,415 to Brassart, et.al. (Nestec S.A. CH); 5,792,754 to Green et. Al. (Nutricia NL). Of most relevance is U.S. Pat. No. 5,721,345 to Roberfroid, et. Al. titled Prevention Of Mammary Carcinogenesis and Breast Cancer Treatment. U.S. Pat. No. 5,550,113 to Mann titled Blood Sugar Regulating Composition and Methods Relating Thereto. Also disclosed is a method for regulating blood sugar levels by administration of the composition of this invention.

The category of soluble fibers called gums has been previously mentioned. Gums are a natural addition to functional foods. Not only can they improve the appearance, stability, and the overall appeal of your finished product but they are also a valuable source of dietary fiber.

Of specific interest here, and to be included in the invention, is to use gums with known functional properties. Literature supports the following: Guar gum improves insulin sensitivity, blood lipids, and blood pressure. American Journal of Clinical Nutrition 56:1061-5; 1992. A study at the University of Minnesota confirmed that with the inclusion of guar gum the viscosity of the contents of the intestines was increased.

Further Benefiber ® by Novartis is a guar gum used for the treatment of diarrhea.

Gum Arabic has been shown to enhance water, electrolyte, and glucose absorption from oral rehydration solutions in jejunal perfusion of healthy rats and in animals with theophylline-induced secretion or chronic osmotic secretory diarrhea. Gum Arabic while having perhaps a little effect on glucose tolerance and stool weight has been shown to have a positive effect on decreasing serum cholesterol.

### Other Active ingredients

Other than soluble fiber the inventor specifically favors active ingredients, which permit the creation of a beverage containing active ingredients for which there exists sound science. U.S. Pat. No. 5,981,498 to Fukuda, Miyake (KKHSKK Okayama, JP) titled Agent for improving blood circulation. U.S. Pat. No 6,008,252 to Beale titled Method for increasing muscle mass. U.S. Pat. No. 5,888,514 to Weisman titled Natural composition for treating bone or joint inflammation. U.S. Pat. No. 5,716,976 to Bernstein titled Method of treatment for carbohydrate addiction. U.S. Pat. No 5,820,867 to Bewicke titled General antidepressant composition for dietary supplement. U.S. Pat. No to Tomer (Cromak Research N.J.) titled Herbal composition and their use as hypoglycemic agents. U. S. Pat. No. 5,741,491 to Jones (Isotechnika Inc. CA) titled Medicinal composition for diabetes. U.S. Pat. No. 6,025,363 to Giles titled Composition for suppressing appetite.

It is critical to this invention that each packet be consistent in containing the represented amount of active ingredients. It is difficult at best in production to weigh out ingredients especially if they are of varying weights. U.S. Pat. No. 4,959,947 to Reif (Motan Verfahrungstechnik GmbH & Co. Weingarten, DE) teaches an apparatus for the production and packaging of a compound mixture, in which extremely accurate and rapid weighing-out, proportioning and packaging of individual components are achieved.

The invention set forth here includes, one or more functional and or active ingredients which functions independently and or in combination with true tea and or one or more herbal constituents and or any combination of the aforesaid. As example the inventor is most focused on dietary fiber, antioxidants, pro-biotics, pre-biotics, enzymes, etc. and conceives of them to be added, one or more, and in any combination maintaining the integrity of the aforesaid.

### Antioxidants

Antioxidants help to protect the body from the formation of free radicals. Free radicals can cause damage to the cells, impairing the immune system and leading to infections and various degenerative diseases such as heart disease and cancer. Free radical damage is thought by scientists to be the basis for the aging process as well.

The following antioxidants are a consideration of the inventor:
1. Alpha-Lipoic Acid helps to neutralize the effects of free radicals.
2. Bilberry is a strong antioxidant that keeps capillary walls strong and flexible. Supports and strengthens collagen, inhibits growth of bacteria, anti-inflammatory, anti-aging, anti-carcinogenic.
3. Coenzyme Q10 is an immunologic stimulant, increases circulation, anti-aging, and beneficial for cardiovascular system.
4. Cysteine (also an amino acid) detoxifier of alcohol, tobacco smoke, and environmental pollutants. Anti-aging.
5. Glutathione defends against damage from smoking, exposure to radiation, cancer chemotherapy, and toxins such as alcohol. Detoxifier of heavy metals and drugs, aids in the treatment of blood and liver disorders.
6. Melatonin is an antioxidant/free radical scavenger.
7. Selenium guards cells of blood, heart, liver, and lungs. Stimulate antibody response to infection.
8. Vitamin C free radical scavenger. Increases the synthesis of interferon (natural antiviral substance produced by the body).
9. Vitamin E prevents the oxidation of lipids.

Other antioxidants include but are not limited to Green Tea, Grape Seed Extract, Superoxide Dismutase (SOD), Vitamin A and Beta Carotene, and Zinc. The inventor envisions formulations of one or more antioxidants in combination with any other ingredient to be said or aforesaid.

### Biotics

The basic formulations for the infusion packets will be built around the groups commonly known as fiber, pre-biotics, pro-probiotics, antioxidants, and amino acids, and digestive enzymes. The inventor, while believing in vitamins and minerals and the need for both groups, on a daily basis has concerns that there are to many products out there today which are pushing these that there is concern about getting too much. While the inventor has a great respect for herbal constituents best in these formulations to use them for flavoring, color, aroma, and not to rely on them as active ingredients. Again because there are too many products in the market today, and more because of the inability to standardize and or know the quality, potency etc. of each batch.

Probiotics are organisms and/or substances which help to improve the environment of the intestinal tract. Probiotics are foods that contain live bacteria and are known to increase milk digestibility, speed recovery from diarrhea, enhance immune function, reduce certain cancers, and lower of blood cholesterol levels.

Prebiotics are foods or nutrients that are used by specific bacteria and that can be added to the diet to increase the chances of these particular bacteria growing and thriving in the intestine.

The bacteria that live in the intestines make up a very large and very diverse population. The numbers of each kind of bacteria change depending on age, diet, health status, and use of drugs and supplements. The bacteria that do thrive do so because they are able to adhere to the intestinal wall and use the semi-digested food that is passing through the intestines. Because some bacteria have specific nutrient requirements it has been proposed that adding these particular foods or nutrient to the diet could be a way of increasing the numbers of specific bacteria.

U.S. Pat. No. 6,180,099 to Paul, (Metagenics), titled, "Method Of Using Immunoglobulin And Fiber-Containing Compositions For Human Health" identifies preferred and beneficial human intestinal microorganisms such as *Lactobacillus acidophilus, L. bulgaricus, L. casei, L. fermentum, L. salivaroes, L. brevis, L. leichmannii, L. plantarum, L. cellobiosus, Bifidobacterium adolescentis, B. infantis, B. longum, B. thermophilum,* and *B. bifidum.* More preferably, the beneficial human intestinal microorganism is selected from *L acidophilus* and B. *adolescentis.*

Probiotics and prebiotics are delicate and sensitive and up until recently, have not been able to be handled effectively long-term, without refrigeration. With the new microencapsulation technologies available today, we are now able to handle these organisms successfully. Not only are they valuable in human health but animal health as well.

### Enzymes

The late Dr. Edward Howell, a physician and pioneer in enzyme research, called enzymes the "sparks of life". These energized protein molecules play a necessary role in virtually all of the biochemical activities that go on in the body. They are essential for digesting food, for stimulating the brain, for providing cellular energy, and for repairing all tissues, organs, and cells. Life as we know it could not exist without the action of enzymes, even in the presence of sufficient amounts of vitamins, minerals, water, and other nutrients.

Enzymes are often divided into two groups: metabolic enzymes and digestive enzymes. It is the later that we shall concentrate on for the most part in the infusion packet formulations. Digestive enzymes are secreted along the gastrointestinal tract and break down foods the nutrients to be absorbed into the bloodstream for use in various bodily functions. There are three main categories of digestive enzymes: amylase, protease, and lipase. Amylase, found in saliva and in the pancreatic and intestinal juices, breaks down carbohydrates. Different types of amylase breaks down carbohydrates. Different types of amylase break down specific types of sugars. For example lactase breaks down milk sugar (lactose), maltase breaks down malt sugar (maltose), and sucrase breaks down cane and beet sugar (sucrose). Protease, found in the stomach juices and also in the pancreatic and intestinal juices, helps to digest protein. Lipase, found in the stomach and pancreatic juices, also present in fats in foods, aids in fat digestion.

Digestive enzymes are very important on a regular basis. While the body manufactures a supply of enzymes, it can also obtain enzymes from food. Unfortunately, enzymes are extremely sensitive to heat. (Even low to moderate heat (118 degrees F (48 degrees C) or above) destroys most enzymes in food, so to obtain enzymes for food one must teat raw foods. Unfortunately the eating of raw food is not prevalent in out society today. Research has shown that as we grow older, the body's ability to produce enzymes decreases. At the same time, mal-absorption of nutrients, tissue breakdown, and adverse health conditions increase.

The alternative is to take enzyme supplements, which reduce the stress on the body etc. Today digestive enzymes are available over the counter in tablet, liquid, capsule, form.

It is the object of this invention to provide digestive enzymes, separately and/or in combination with each other. If they are microencapsulated there is a greater potential for stability and potency. Further they may be combined with other ingredients to compliment such as peppermint which is know to be good for digestion.

### Amino Acids

Amino acids are the chemical units of protein. Assuming the reader knows the importance of protein it is not necessary for the inventor to elaborate. It is only relevant from which to go forward. Proteins are amino acids linked together to form peptide bonds. Each individual type of protein is composed of a specific group of amino acids.

In addition to combine to form the body's proteins, some amino acids act as neurotransmitters or as precursors of neurotransmitters. Further, amino acids enable vitamins and minerals to perform their job properly.

For certain effects and certain disorders, taking supplements of specific amino acids can be very beneficial. When taking a specific amino acid or amino acid combination, it supports the metabolic pathway involved in a particular situation. Vegetarians, especially vegans, should consider taking a formula containing all of the essential amino acids to ensure that there protein requirements are met.

The inventor here forth presents a brief description of some of the amino acids. However in contemplating the contents of the infusion packet invention one or more may be included. The chemical structure will be the L form. The delivery system used will be the most compatible to ensure potency and stability while realizing that the amino acids are stable at room temperature and decompose when heated to temperatures of 180 degrees C to 350 degrees C.

With the information presented below it will be obvious to see the possible combinations of fiber, amino acids, antioxidants, etc. and the like in multiple formulations.
1. Alanine aids in the metabolism of glucose.
2. Arginine retards the growth of tumors and cancer by enhancing immune function. May benefit those suffering from Aids and malignant diseases that suppress the immune system. Good for live disorders, cirrhosis, fatty liver, detoxifying. Useful in treating sterility in men. Important for muscle metabolism. Aids in weight loss because it facilitates an increase in muscle mass and a reduction in body fat. Stimulates the pancreas to release insulin.
3. Asparagine maintains balance in the central nervous system (CNS).
4. Aspartic Acid increases stamina, it is good for fatigue and plays a vital role in metabolism. Good for athletes. Beneficial for neural and brain disorders.
5. Carnitine is more a substance related to the B vitamins. Main function is to help transport long-chained fatty acids, burned within the cells to provide energy. Major source of energy for muscles. Increases the use of fat as energy.
6. Citrulline promotes energy, stimulates the immune system.
7. Cystein and Cystine helps to detoxify and protect the body from radiation damage. Good for rheumatoid arthritis, hardening of the arteries and cancer. Promotes healing after surgery and severe burns, chelates heavy metals. Promotes the burning of fat and promotes the building of muscle.
8. Dimethylglycine (DMG) helps the body maintain high energy levels and boosts mental acuity. Has been found to enhance the immune system and to reduce elevated blood cholesterol and triglyceride levels. Improves oxygen utilization by the body, helps to normalize blood pressure and blood glucose levels. May also be useful for controlling epileptic seizures.
9. Gamma-Aminobutyric Acid acts as a neurotransmitter in the central nervous system. Can be taken to calm the body. Used in treatment of epilepsy and hypertension. Good for depressed sex drive and also useful for enlarged prostate, probably because it plays a role in the mechanism regulating the release of sex hormones. GABA is effective in treating attention deficit disorder.
10. Glutamic Acid increases the firing of neurons in the central nervous system. It is important in the metabolism of sugars and fats. Helps to correct personality disorders and is used in the treatment of epilepsy, mental retardation, muscular dystrophy, ulcers, and hypoglycemic coma, a complication of insulin treatment for diabetics.
11. Glutamine promotes mental ability and the maintenance of a healthy digestive tract. Helps to build and maintain muscle, supplemental glutamine is useful for dieters and bodybuilders. Can be helpful in the treatment of arthritis, autoimmune disease, fibrosis, intestinal disorders, peptic ulcers, and connective tissue diseases.
12.Tyrosine is a precursor of the neurotransmitters norepinephrine and dopamine, which regulate mood, among other things. Tyrosine acts as a mood elevator. It suppresses the appetite and helps reduce body fat. It aids in the production of melanin (the pigment responsible for skin and hair color). Supplemental L-Tyrosine has been used for stress reduction. Has been used to help individuals suffering from anxiety, depression, allergies, and headaches as well as persons undergoing withdrawal from drugs.
13.Valine has a stimulant effect. Needed for muscle metabolism, tissue repair, and the maintenance of a proper nitrogen balance in the body. Good for correcting the type of severe amino acid deficiencies that can be caused by drug addiction.

Of course, additional amino acids can also be included as needed.

The packet may be sized to fit within a specific container that holds the proper volume of liquid. This may be portioned in single or multiple servings. If used for animals so thus proportioned. Each infusion packet should be proportioned to approximate a specific quantity of liquid. An added advantage of this kind of a delivery system is that the consumer may strengthen and or weaken the solution by reducing and or adding more liquid during preparation.

The inventor has taken note that with all the billions of bags produced over time no one has colored a fabrication for an infusion packet, including and/or not including a connecting device to a tag and/or the tag itself, and/or colored a packet per se by using colorings with the unique nature whereby the colors may change based upon coming in contact with moisture and or governed by changes in light and or heat independently and or simultaneously.

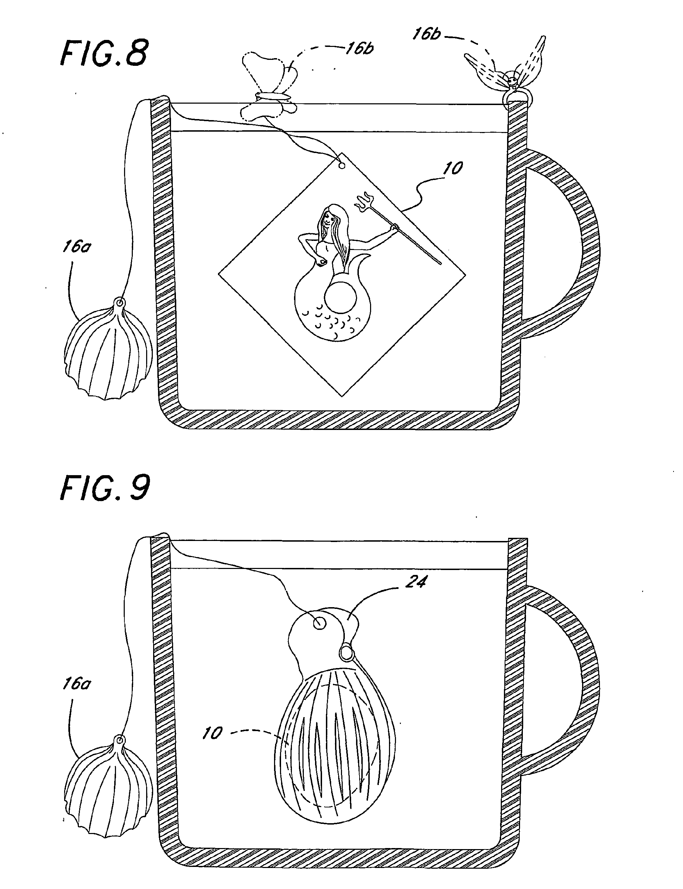

The colors contributing to the liquid exclusively may contain non-active and or active ingredients, which, may when wetted, may totally and/or in part contribute color and/or color and additional active ingredients to the final liquid be they nutritional, flavorful, and/or fragrant liquid beverage and/or soup. An appropriate example is a red and/or a red-orange pigment made from the antioxidant lycopene found in tomatoes and watermelon. U.S. Pat. No. 5,965,185 to Hartal, Raveh, Wolf, (IL), titled Stable lycopene concentrates and process for their preparation. U.S. Pat. No. 5,993,880 to Frost & Saleeb (Kraft Foods Inc. IL) teaches a new form of green color prepared by specially treating copper chlorophyllin. The art further recognizes the use of vegetable extracts as colorants and additives to the food industry. U.S. Pat. No. 4,851,339 to Hills titled Extraction of anti-mutagenic pigments from algae and vegetables. U.S. Pat. No. 5,019,405 to Sapers, titled Process for manufacture of maraschino cherries in a dyeing process using cartenoids. U.S. Pat. No. 4,853,235 to Tomomatsu. (The Quaker Oats Co. IL) Color changing cereals and confections. Taught here the ability of a cereal and/or a confection to change color when immersed in an aqueous medium. For example, Figs. 8 and 9 illustrate a liquid induced color change in the context of the present invention.

In all instances the packet is designed to allow the penetration of/and into liquid (mainly water) either temperature specific or non-temperature specific as determined by the contents. The nature of the ingredients may serve as a guideline as to the temperature of the liquid preferred so as to maintain the integrity of the ingredient(s). In the case of a child one would want to refrain from using potentially burning hot water and thus would want the ingredients to be readily dispersible in a comfortable to the touch liquid. This may also be most important for seniors and or those with any difficulty handling seriously hot water either by mouth and or when mixing.

When mixing in other than hot water it is necessary that the ingredients totally dissolve and dissolve rather quickly. All is with the understanding that the openings are selected so that the contents are effectively retained. An example of the just mentioned would be the products Kool Aid® and Crystal Lite®.

This then deviates totally from the usual botanical material of an infusion packet whereby the materials contained are leaves, flowers, petals, buds, stems, roots, etc. U.S. Pat. No. 5,965,162 to Fuisz, Cherukuri, Kota, Suresh, Stewart, (Fuisz Technologies Ltd. VA.), process for preparing comestible units which disperse quickly in the mouth using fuse and compression technology. Ingredients may be in crystallized form. U.S. Pat. No. 5,866,188 to Batist, Meyers, Fuisz, (Fuisz Technologies Ltd. VA), titled Comestible composition having spheroidal crystal sugar. U.S. Pat. No. 5,549,757 to Morano, (Ingredient Technology Corporation Innovative Sweeteners (NJ), titled Process for recrystalilizing sugar and product thereof.

To make product for inclusion in the infusion packets the inventor is aware of the agglomeration process and additionally the lyophilization process. U.S. Pat. No. 5,051,269 to Noreille, Pot Nestec S.A. (Vevey CH), titled Agglomeration method. U.S. Pat. No. to Stipp, (The Proctor and Gamble Co. ,OH), titled Infusion beverage product comprising co-agglomerater creamer and sweetener suitable for bag and filter pack brewing. U.S. Pat. No.5,616,355 to Haast, Harrell, titled Lyophilized health food products and methods of making same.

The presentation could include an additional supplemental product, also a food which could be, by virtue of the ingredients, supplemental to the ingredients in the packet. The inventor is most interested in the inclusion of active ingredients in confectioneries (candies). U.S. Pat. No. 5,928,664 to Yang, Oh, ( Fuisz Technologies Ltd., VA), titled Consumable gummy delivery system teaches also the inclusion of active ingredients admixed with a glycerated gelatin matrix. U.S. Pat. No. 5,578,336 to Monte, (AZ), titled Confection carrier for vitamins, enzymes, photochemicals and alimentary vegetable compositions and method of making.

It is also contemplated that inside the infusion packet can be placed a gummy candy and or anything resembling a confection. The only requirement is that it dissolve totally and or in part to make a beverage. This then becomes a significant part of the invention whereby a consumable delivery system for active ingredients comprising of a plurality of candy composition carriers comprising a plurality of active ingredients, whereby the composition is enclosed in an infusion packet. This could resemble one or more gummy bears in a packet. Additional gummy bears, by example just mentioned, could be provided with the infusion packet. These can be in the same wrapper, and/or the same container. Here is where the parent and/or caregiver can encourage the child to make the beverage, watch the bear dissolve and as the reward get the extra few candies. The extra few candies, as aforementioned, also carry active nutritive ingredients. An important aspect of these candies, which must be emphasized, is that they will include one or more fibers, contributing to the daily need for fiber by everyone. These fibers, especially the gums, will also add to the mouth-feel.

### Flavors and Encapsulations

While it is possible to make attractive looking products, one also needs to factor in taste. The inventor is most sensitive to the fact that many ingredients which are good for you do not taste good. This shall be addressed in more detail under encapsulations which may or may not include liposomes. Meanwhile the inventor has taken special note of the following U.S. Pat. No. 5,927,052 to Nippes, Klein (Teepak, Meerbusch, DE) titled Method and device for flavoring tea and tea-like. U.S. Pat. No. 6,022,576 to Cirigliano, Farrell, McKenna, Thomas, Rothenberg (Lipton C., NJ), titled Flavoring materials for use in tea containing beverages. U.S. Pat. No. 4,851,252 to Greither, (Bruchkmuhl, DE) titled Composition and process for the production of a mixture for a tea drink with fruit. U.S. Pat. No. 5,633,027 to Cherukuri, Battist, Zamudio-Tenna (Fuisz Technologies Ltd., (VA) titled Confectioneries containing stabilized highly odorous flavor component deliver systems.

Micro-encapsulation is the process of enveloping certain drugs, enzymes, and the like in polymeric matrices designed to be used in controlled release or delayed release applications.

The International Micro-encapsulation Society, founded in 1995, Glasgow, is dedicated to foster and promote communication and collaboration between amongst science professionals. They define micro-encapsulations as a process that allows liquid or solid substances to be covered by a barrier wall. The wall must be chemically inert to the content of the capsule and possess an adequate stability to mechanical, thermal, or chemical influence. Various barrier wall materials may be utilized during encapsulation which are dependent upon the application.

Encapsulations, which release their contents within the packet so that the released active is transported across the membrane, may be included. Alternatively the entire encapsulation pass through the membrane intact and release in encapsulated active ingredient into the infused liquid either before or after ingestion of the infused liquid. Additionally, the encapsulations may be timed released, and even on an individual basis of each encapsulation so that the ingredient(s) may be released in the mouth or anywhere in the digestive tract within a period of one to three hours after ingestion. Further the encapsulations under the aforementioned time release may be totally contained within the membrane of the packet, partially contained within the membrane in any ratio desired of packet to interior ingredients. This would fall under what the inventor calls "best use" of active ingredients. This can also apply to color encapsulations and or aroma encapsulations as to the formulas just mentioned and to those skilled in the art of.

The Wurster process is a coating technique that is well suited to uniformly coat or encapsulate individual particulate materials. The Wurster process is an internationally recognized coating technique for precision of film coat to particulate such as powders, crystals, or granules.

The coating of pharmaceutical and or nutraceutical micro-encapsulations helps ensure and optimize stability and prolong shelf life of reactive ingredients. Capsules may be coated for improved barrier properties. Coating is a most effective way of masking the taste or odor of a particular ingredient, making products more palatable. Enteric coatings can be adjusted most easily to controlled and or timed release for the maximum health benefits. In the food industry micro-encapsulations are beneficial especially to encapsulate vitamins, minerals, and functional food ingredients. Thin or partial coatings are very effective in reducing the caking of certain materials.

For example, Bio Dar was established in 1984 as a United States and Israeli joint venture now under LycoRed, Koor Group of Companies. They are specialists in microencapsulated vitamins and minerals for the fortification of food products. Their specialty extends to technologies of how to keep the food additive particle from imparting an undesirable taste to the surrounding ingredients. This technology is most valuable for where the micro-encapsulations are mixed in with the other ingredients. Further they deal with Carnetine, Amino Acids, Herbal Extracts as well as other nutritional components where the role of micro-encapsulation is to avoid hygroscopicity, minimize interactions and eliminate the oxidation of these materials. Further they have the ability to do multiplayer micro-encapsulations for mainly drug delivery. U.S. Pat. No. 4,749,575 to Rotman, (Bio-Dar Ltd. IL), titled Microencapsulated medicament in sweet matrix.

Other patents of reference are US 4,711,784 (Yang/Warner Lambert; 5,024,842 (Edgren, Theeuwes/Alza Corp.); US 5,051,261(Mc Ginity, Chang/FMC Corp); US 5,009,819 (Popescu, Mertz/The Liposome Company); US 5,653,996 (Hsu/Genentech CA); US 5,891,465 (Keller/Bio-Zone Laboratories Inc CA); US 6,007,838 (Alving, Owens, Wassef, Nabila (U.S.A. Dept. of the Sec. of the Army/ Washington D.C.); US 6,190,591 (van Lengerich/General Mills MN); and US 5,922,350 (Janoff et. AI./ The Liposome Company NJ).

The inventor is not aware of the previous use of encapsulation technology to augment infusion packets. This technology allows the combination of hitherto incompatible ingredients so that infusion packets can readily deliver a variety of active agents including stabilized enzymes and similar labile substances. In some cases the encapsulations can be designed to simply dissolve or burst and release their active contents right into the infused liquid. In other cases the encapsulations can be designed to leave the packet intact and exist as colloidal particles suspended within the infused liquid. Such suspended capsules can then release their contents at the correct position within the digestive system of the person consuming the infused beverage. Thus, an enzyme or ingredient sensitive to stomach acid would be released in the intestine to do its work in the proper milieu.

Today, 70 million Americans suffer from digestive diseases, 15 percent on a daily basis (NIDDK 1997). An even larger population, approximately 118 million, experience heartburn or are afflicted with gastroesophageal reflux disease (GERD) at least once a month. Even more potentially alarming is the projected 35 percent increase in the number of adults 50-64 who will be afflicted with digestive problems. It is estimated in America that 90 million people use antacids or other stomach relief medicines (Euromonitor, 1998). Next to headaches stomach problems are one of the most self treated ailments in the U.S. (American Pharmaceutical Ass., 1997) Further one of the strongest health links for nutraceuticals and treat digestive problems.

The inventor believe that much of these problems are caused by overly acidic beverages over used over long periods of time. Further as we age we loose our ability to produce not just quantity but a good quality of digestive enzymes so necessary to break down foodstuffs.

It is the goal here to provide a healthy answer to the above by using well researched ingredients, non acidic ingredients, and vehicles such as encapsulations regardless of size and/or of materials, including but not limited to liposomes, to address and support digestion along with promoting a healthy digestive tract environment.

### Tether

Although the packet may be simply thrown into hot water and later retrieved with a spoon or other appropriate tool, the packet often has an attached string 13 or similar device so that the packet can be "tethered" and readily recovered at the end of the infusion period. U.S. Pat. No. 4,609,556 to Goedert presents a filter bag adapted to be maintained by means of an ordinary spoon in order to keep the bag from floating to the top and or more easy to remove. It is here conceived in this invention that at the end of this string maybe positioned a loop to go around the finger, especially for a child, said loop may be attached to a coil like member as opposed to a string. Or, it is possible to attach a ring with a diamond like stone to the end which can be removed and kept by the child afterwards.

The inventor believes that removing the packet can be especially messy as it is wet and likely to drip. Disposing of it may also be problematic, as well as inconvenient. Especially noteworthy is if the consumer is "on the go" in the car, at the gym, at a sporting or entertainment event as just presenting a few examples. To address the just mentioned it is the intent of this invention to provide a solution, and a solution with more than one value. It is understood by the inventor that the size, height of the bottles change due to the volume of contents inside. To that end a taller bottle would require a longer string (e.g. tether between the infusion packet and the external tag). Therefore a longer string would be provided so that there is ample length for the infusion packet to sit on the bottom of the bottle and, additionally, provide ample length to extend outside the bottle to at least no less than the midline of the bottles exterior. As an example using a 50.7 oz. (1.5 I) bottle, with a normal height of 12 inches (30 cm), one would provide a string no shorter than about 18 inches (45 cm).

The tag of the bottle embodiment of the current invention can be made to any size, decorative, etc., but additionally carries with it with an adhesive backed material paper etc. by which to attach the tag to the outside of the bottle. As example 3M's Packaging Systems division offers many options for attachment packaging. In this fashion the consumer does not have to remove the packet nor fiddle with a hanging string.

While "scratch and sniff' technology has been used for years so has the ability to impregnate materials with ingredients by using special mechanisms of release, deliver to a specific cause/site fragrance. Further is conceived the ability to provide an aromatic experience for the consumer. Most noteworthy and , also a 3M development, is their trans-dermal and or trans-mucousal delivery systems. Based on that technology 3M has developed a technology by which to impregnate a material with aroma. This is done by protecting the material with adhesive or in the layering of adhesive material itself imbedding fragrance. The aroma is would be released when pealing off the protective portion of the adhesive. The object here is to provide an aromatic experience for the consumer other than what emanates from the beverage inside. This invention then obviates the disadvantage of not having an open beverage container whereby the scent may more readily be apparent. Energy Brands of CT has fruit-water products in wide mouth bottles so the consumer may smell the essence as well. U.S. Pat. No 5,885,640 to Anderson, (Nestec S.A. Vevey CH), teaches a package which provides for an aromatized head space by mixing the aroma with gas. In this case Nitrogen would be an excellent choice.

This aroma will serve to enhance the flavor of the beverage by mirroring the ingredients (cherry, lemon, orange) in the packet and or compliment the ingredients (chocolate aroma to a cherry flavored beverage) or any combination thereof while, at the same time, securing the tag to the outside of the bottle.

The placement of an aroma tag is up to the consumer's nose. If the tag is close to the opening, even placed in alignment with and/or over the screwing groves, the more available the scent is to the nose of the consumer.

### Support Members

Through out history, the world, and existing in multiple cultures there are rituals centered around eating drinking, smoking, (i.e. the Indian peace pipe, marijuana, etc.). While the fork is considered by most to be a more advance eating tool than chopsticks, chopsticks are still used. Additionally the inventor has personally seen the delight, especially with children, who attempt to eat in this fashion.

We are a paraphernalia, gadget, technology driven society, which seems to be thriving on interactive experiences of every kind. To that end the inventor, in an effort to attract consumers and direct them towards more healthier drinking, chooses to set forth as part of this invention a combination of an infusion packet and/or packets with a support member. This is of special appeal to easily impressionable children and "me too" teenagers.

To this invention a single packet may have its own support member. Further, one support member maybe included in a container with more than one packet. This support member can facilitate ease of use, may serve solely as entertainment, a part of a promotion, a contest, or advertising, etc. and/or any combination of the above.

There are a variety of analogs to the support member that are currently used by the confectionery industry to deliver more product to the consumer in an entertainment and/or and "edutainment" like fashion. It is of great interest to note how the confectionery industry has incorporated the use of toy or toy-like components as part of a dispensing mechanism. Overtime one of the most easily recognized candy dispensers was the PEZ® container. This is a little pocket container that contains small rectangular sugared candies that when activated brings forth one candy at a time. Bondi, a Japanese toy company, has used a variation of the PEZ® theme using Pokamon in the same fashion.

Today, there a numerous and quite elaborate candy and gum ball dispensing machines. These are mainly manufactured for use by the very large candy companies such as Mars. These machines deliver candies such as M&M's,. Many of these machines have licensed characters on them. Additionally, battery operated lollypop holders are most popular. The handle turns the lollypop. Further the handles are often fashioned after popular animated characters. U.S. Pat. No. to 5,690,535 to Coleman, T.; Schlotter, W.; Coleman, P.A.; Schlotter, A; entitled Twin Spins Spin Pop. Also to the same inventors U.S. Pat. No. 5, 676, 988 entitled U.F.O. Pop.; and U.S. Pat. No. 5,820,437 titled Wacky Pop Noise Maker. U.S. Pat. No. 5,542,570 to Nottingham (Cap Toys Inc.) titled Toy Dispenser with Feed Means. U.S. Pat. No. 5,862,997 to Coleman titled Pop-eye Pen and Candy Holder. U.S. Pat. No. 5,897,022 to Mann titled Kinetic Activity Gumball Dispensing Device. The object of all of these is to capture the consumer combining entertainment with the treat. That is, it is within the concept of the support member of the current invention to have a support member with "glow in the dark" or moveable parts, with a battery or other power to provide movement, light or sound.

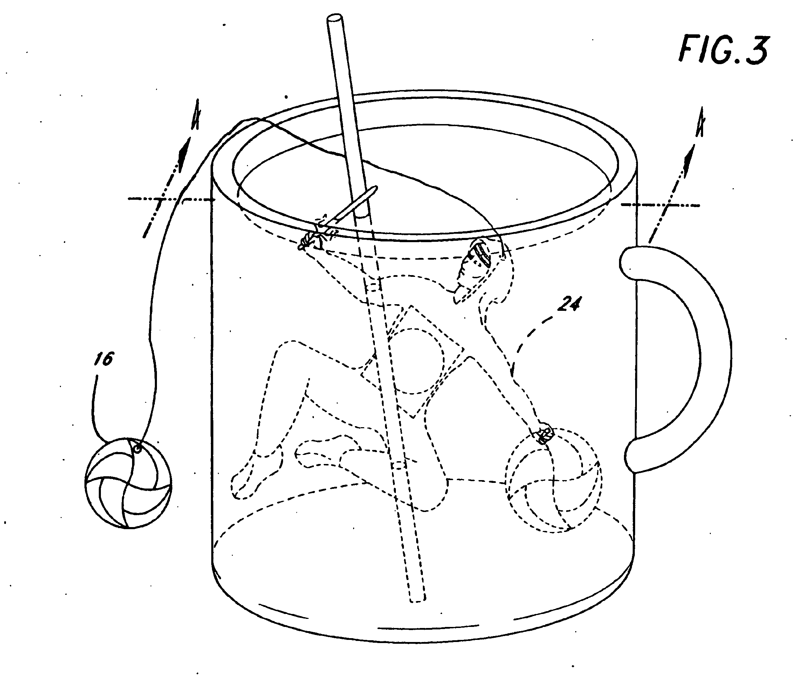

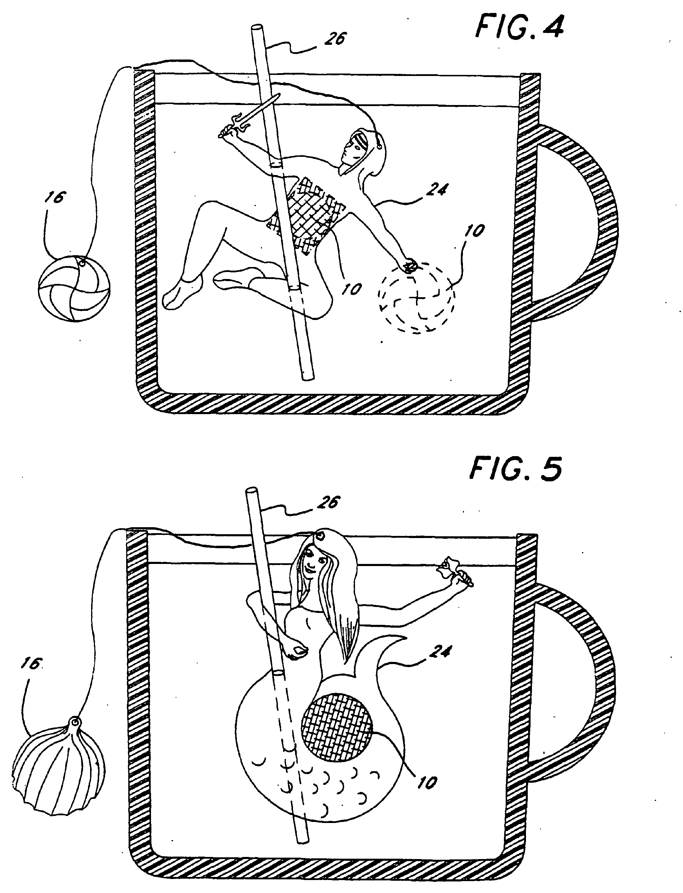

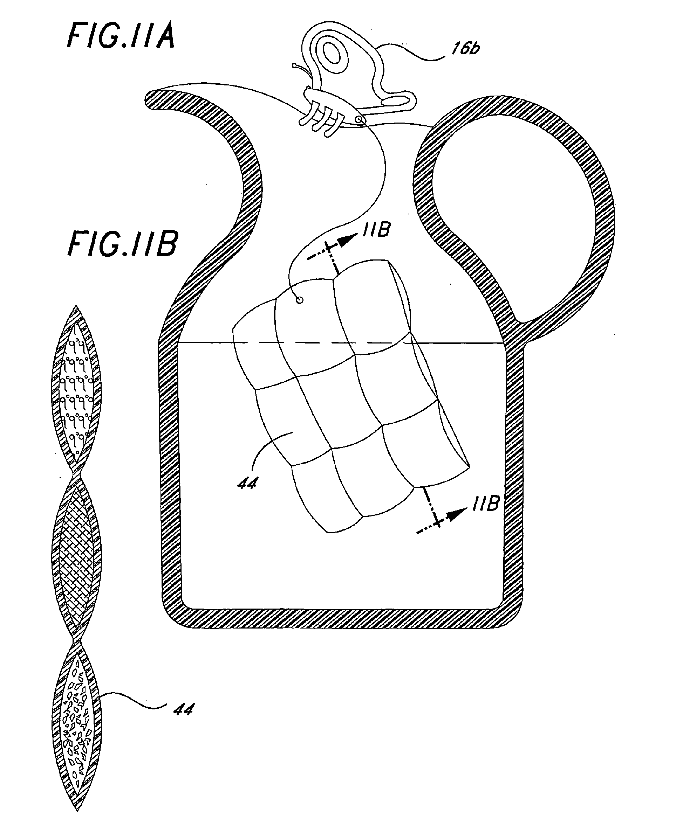

The infusion packet 10 may or may not be attached to the support member 14. If it is attached then there could be one support member for each packet. That is the support member may surround the packet as in Fig. 3 (also Figs. 4 and 5). In addition, as shown in the figure, the support member can also serve to support a drinking straw 26. Fig. 11 shows an alternate embodiment where the support member is used to join individual infusion packets..

If it is not attached then one or more support members may be in the container and be used interchangeably. That is, a support member may act as a tool to handle or immerse the diffusion packets. Such a tool may in itself be ornamental and function as jewelry, etc. Further it is possible to supply many packets so as to let the consumer use more than one on a support member to, in effect, create unique drinks by personal design.

In the beverage market there exists products, which have these characters on the aseptic box packaging system, the bottle, or can but nothing more. It is here forth a part of this invention to include any number of support delivery systems, powered by batteries or not to be used with one or more infusion packets in an effort to encourage "healthy drinking" especially in children.

It is also known that when one is sick, especially a child, it is most difficult to get them to drink at all, yet alone a healthy beverages. To that end not just a single packet but there be fashioned an entire day supply of infusion packets especially designed to address the common ailments of school age children (colds, sore throats, colds etc.). Best method would be to make the presentation part of a toy, game, etc. whereby the child would be encouraged to drink one packet after another. Secret doors in a box may be opened one by one revealing clues as to solving a mystery. The object would be to make a beverage get a clue. Or the infusion packet could have a secret message on it that doesn't become apparent until submerged in water when the whereby the writing becomes visible.

### Tags

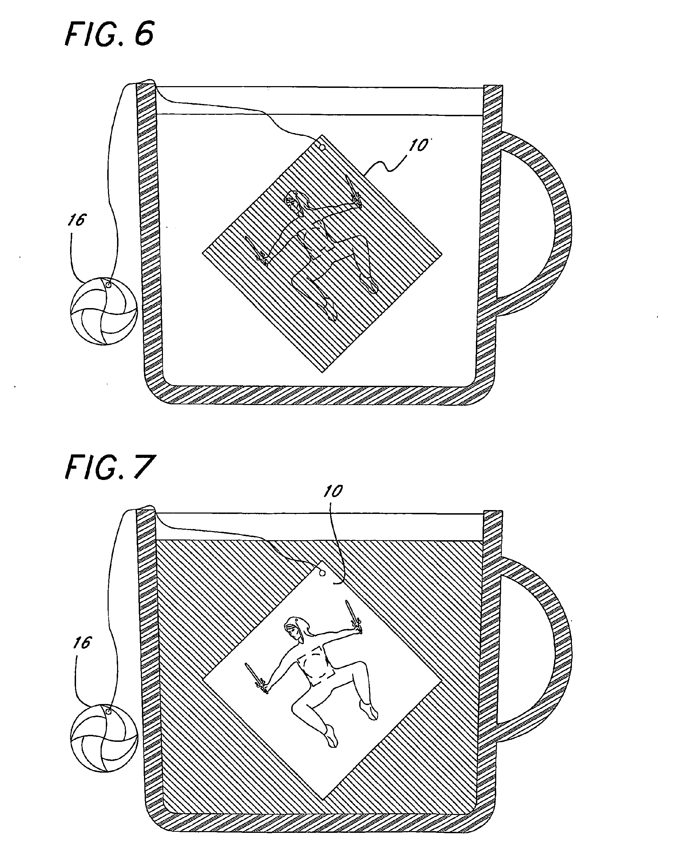

Tea bags and other infusion bags are often provided with a tag 16, attached to the bag itself through a thread 13, to make it more convenient for the user to handle the bag. Just as there is a tag so there lies an art in attaching the string to the tag U.S. Pat. No. 5,580,408 to Vernon, Goodwin, Cleall, (Thomas J. Lipton Co. NJ) titled Method for the production of tagged articles. However, the inventor has taken note that while many tags have printed material on them none have reflected a new additional use and or function. Therefore conceived by the inventor, the tea tag (regardless of construction material(s)) may be of a special shape such as a puzzle piece. Thus in this instance collecting all the tea tags from the infusion packets in one container would fit into a form for a puzzle. That form would be included in the box. These objectives are most appealing to children and to there parents who are trying to guide and or alter their children's drinking choices towards a more healthy direction. Additionally puzzle components etc., and/or the like, may be attached to the packets and/or included with the packet enclosed within the outer wrapper. These could be given out by a major restaurant chain such as Mac Donald's. Therefore every time purchased a liquid (mainly water) the consumer would select a packet of choice. This is much like as if one were selecting a beverage. When one completes the puzzle, game, or have the lucky number ,and/or piece one would be entitled to a reward/prize. This can serve as a contest, a cross promotion, a discount towards a future purchase etc. This is a best effort to hopefully encourage, not just the fast food chains to endorse and supply healthy beverages without having to stock a large ready to drink inventory, but to encourage consumers (mainly children) to "drink healthy". As shown in Figs. 6-7 the tag 16 can form a recognizable part of the regalia of a character (here the ninja pictured on the packet 10). As shown in Fig. 8 the tag 16a can be a charm, or the tag 16b can be a clip to fix the tether to the edge of a vessel. Of course such a clip can also act as a piece of jewelry.

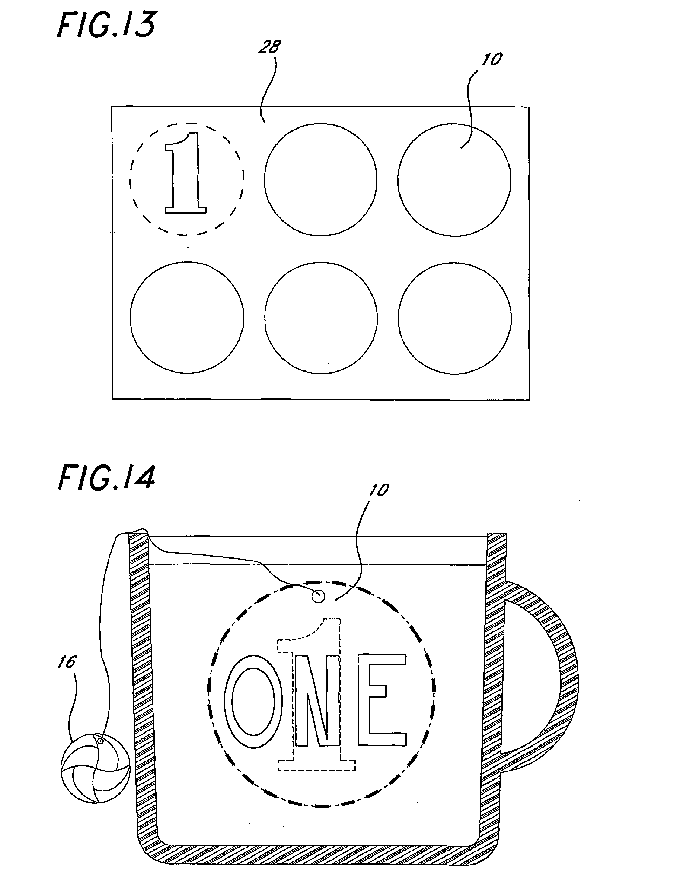

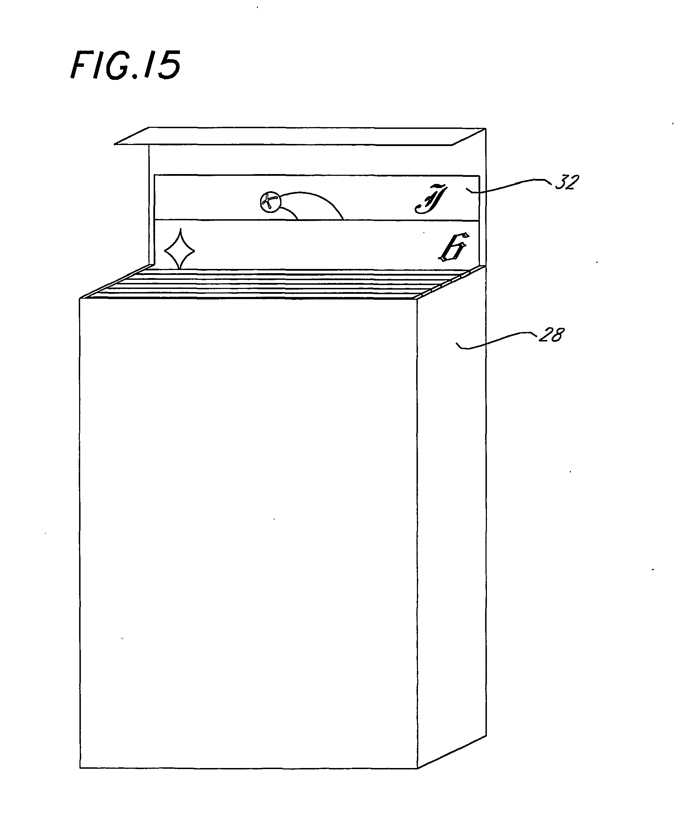

An example of a novel use for tags would be a game such as a card game. The game would consist of a deck of cards usually 52; however, a reduced number could be used-seven of them (as example one for each day of the week). The "cards" would be tethered an infusion packet suspended from each. The aforesaid cards (see Fig. 14) are special to the deck as they represent a wild card, an action card, an instruction card, and/or the like. The object, and here mostly related to a child, is to collect all seven special cards by first using the infusion packet to prepare a beverage, next drink the beverage, and finally dispose of the packet and add the card to the deck. As more cards are added to the deck the game becomes more interesting and challenging.

Further use of space on a tag may include other element of noteworthiness such as printed on lottery numbers, fortunes, quotations, characters, horoscopes, and or the like. As a unit both the packet and or the tag can be printed on in consort. As an example from the child's story Snow White, Snow White can be on the infusion packet and each infusion packet be tagged with a dwarf, or the like. Thus, it is a central part of this invention that the tag serve a special function beyond merely indicating the contents of the attached infusion packet.

Further the tea tag may be made from any material appropriate to serving its function, synthetic or non synthetic. Beyond functionality would be a dual use whereby the tag may be made of a permanent material such as plastic and or metallic foil, so it can be removed from the string and kept. Children love collecting and trading. If well-known characters were used to embellish the tags a "tea tag" fad, could be started, whereby they would become collectable, tradable, redeemable, or serve as a game, and/ or a toy piece etc. Fig. 13 shows a number game wherein the tags 16 show numbers that correspond to numbers revealed in the package 28 when each tag 16 is removed. As an additional "fun" aspect the packet 10 has a hidden version of the same number that is revealed upon immersion. Another game is pictured in Fig. 14. Here a card game is formed from the tags 16, each of which act as cards. The object is to collect a particular "preferential card" 32.

Within the packet itself there can be an assortment of ingredients. Also within a given container of packets there be an assortment of packets which may have not only mixed formulations but also mixed designs of the same theme and/or mixed themes as reflected either on the packet, tag, and/or both. As an example, a container could be totally devoted to one Disney theme with all the characters or a mixed assortment of Disney intellectual properties.

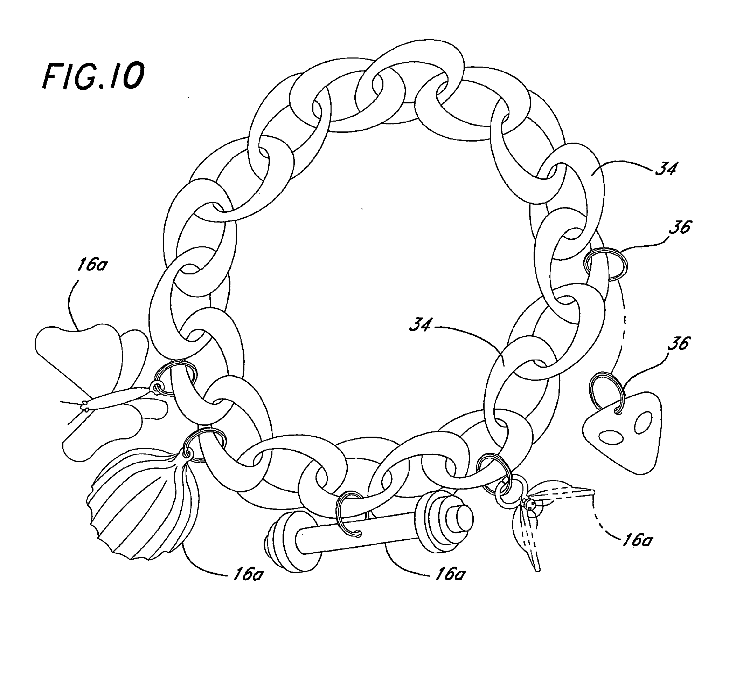

An example of this concept would be to use a container which could contain a plain chain link little bracelet and each tea tag function as a charm, flat or dimensional, so when the thread to the packet is removed the charm/tag maybe attached to the bracelet so as to make a charm bracelet. For this to happen would necessitate an attachment device. To that end a "jump ring" would be provided either on the bracelet or on the tag/charm itself. Fig. 10 shows such a charm bracelet where each tag 16a acts as a charm. The charms can be readily added to a chain loop 34 by means of "jump rings" 36.

These types of situations are most useful in the encouragement of getting children especially to drink the liquid so as to collect the charm. This would present as an appropriate reward to acknowledge and reinforce good drinking as opposed to receiving a sugar-laden reward. It is also conceived that the tag may have a decorative clip on the end instead of a charm. When removed this clip, as illustrated as a butterfly here, when removed could be clipped to clothing and or hair. It is further conceivable that any charm may be fashioned to operate, interchangeably, as a clip or a charm providing the necessary activating parts are present. See Figs. 8 and 10.

### Envelope

In one instance the contents of the packet are enclosed within the folds of a porous paper or non-woven fabric (although woven fabrics are also used) and/or additionally having a synthetic component having a plurality of openings between the constituent fibers and or elements. In a second instance the contents of the packet are enclosed within a structure such as paper, foil, plastic etc., opaque, or transparent (see through) totally or partially having a plurality of openings most commonly known as holes pores, and/or perforations. The openings are proportioned so that the contents are held securely inside until submersion and/or wetted.

Just in all the aforementioned the sheet goods used to fabricate the bag may be colored, printed on, etc. If the specifications call forth for a material that is not porous and thereby holes/perforations are warranted for the transfer and mixing of ingredients then it is conceivable that these openings are made on the sheet goods first, most probably with a die cutting machine. The material may be printed on as well as have the holes made. The holes my be made in any shape and/or collection of shapes, planned or randomly done so long as they support the best use of the invention. It is also possible to have a pattern or design be compatible with the perforations made. An example of this would be to have an alien character that can be submerged where by the liquid is attracted into the bag. Thus when on lifts the bag the liquid can pour out from the alien's features. This again maybe most delightful, and welcomed, by the younger population.

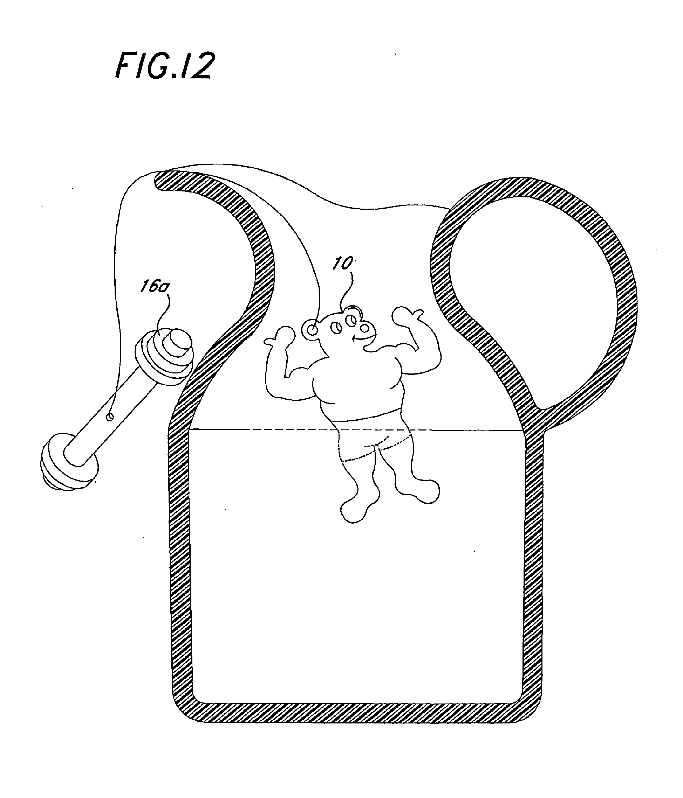

Again, there has been insufficient attention to use of the infusion packet envelope to enhance the overall product. No one has produced a fabrication whereby the surface material is used to display graphics including but not be limited to recognizable symbols and other indicia, cartoon characters, text, advertising materials, representative objects, pictures, jokes, riddles, fortunes, lottery numbers, horoscopes, etc. or the like. The latter is especially of note in regard to children, and to those children who are loyal fans of popular characters such as Mickey Mouse (Disney) Pokamon, and super-heroes such as Superman. Examples of "figure-shaped" packets can be found in Fig. 2B showing the alien figure and matching tag and Fig. 12 showing a packet shaped as a character (bear) 38 with a matching weighted tag 42 to keep the large bear from "flopping" over in the liquid.

In many instances effervescence is desirous or deemed of value. First is may mask the taste of some ingredients. Additionally, it closely resembles soft drinks for which the market is huge.

However working with effervescence is not easy. The inventor is fully aware of the hygroscopic nature of effervescent ingredients, and therefore knows that an outer wrapper must protect it. Upon experimentation the inventor discovered that by having an effervescent tablet inside of a porous bag it floated to the top of the glass, regardless of the temperature of the water. While it did effervesce, it did so at a very slow rate. However, when the tablet was put into a packet with very definite holes, larger pores, not only did it activate faster but it created a *"storm in the tea cup".* The release of the effervescence, and the accompanying bubbles, occurred simultaneously inside the packet as well as outside the packet into the liquid. In some experiments it also moved around quite a bit due to the motion of the bubbles.

With the thought of protecting the effervescence and/or any other ingredients, which are moisture and/or oxygen sensitive, a packet can be made without any openings. Provided with this kind of a packet would be an instrument of any sort by which an individual can make his own holes. This instrument may be provided attached to each individual packet and or be part of the tag. Or it may accompany each individual packet in the same outer wrapper much like a little straw goes with the aseptic juice boxes. Further one or more instruments may be provided in a larger container, such as a box or a tin, that contains many packets. It is also possible to have a design where by the user would automatically be guided as to where to make the holes, like in facial features such as the eyes. At the consumers discretion, the user may decide to make the random holes necessary for activation. With this part of the invention the consumer, in most part, would also control the quantity of material released, and the time involved for releasing would be proportionate also. If there were a tablet inside the packet it would not be problematic if the tablet broke upon puncturing the packet.

An additional part of the invention the use of clear and/or opaque films either water insoluble or water soluble. They each have a purpose in the invention. Further they can be used independently and/or in combination with each other. Many infusion products are packaged in a clear outer wrapper but the infusion packet itself contained within is not transparent. With this invention it is possible to have a clear outer wrapper and a clear encompassing material for the ingredients. The packet material may be water insoluble with the appropriate holes made. Or it may be water soluble meaning that it totally, or almost totally, dissolves when introduced into liquid.

Either the water-insoluble film or the water soluble film may be impregnated with encapsulated ingredients whereby the material may remain clear, or become opaque.

University of California researchers have succeeded in developing water insoluble films by developing a novel procedure using a variety of protein sources to produce a water insoluble films. U.S. Pat. No. 5,543,164 to Krochta, McHugh, (Univ. of Calif.), titled Water Insoluble Protein Based Edible Barrier Coatings and Films. These films can be useful in protecting food products from oxidative degradation and moisture migration. Tests have shown that these protein based films are excellent oxygen barriers, having oxygen permeability very close to plasticized poly(vinylidene chloride) (PVCD) and less than polyvinyl chloride (PVC). The water vapor permeability of the protein barriers is comparable to other based edible films, and they prove to be completely insoluble in water. It is conceivable that an infusion packet could be made of this film with the necessary holes made for the transfer in liquid. These films are clear and would readily display not just the ingredients but make the activation process viewable. It is conceived that this film be used alone and or in combination with the same water insoluble film as the wrapper. As an example, the water insoluble film may be the clear outside covering for a soluble film product and serve as the outside wrapper. The formation of the film is highly flexible and when in use can protect thus improve flavor, aroma, etc. The same materials used together, would have the resemblance of clear, and would allow for complete viewing. Further either the wrapper and/or the packet itself maybe printed on.

Using a water soluble film it is further conceived whereby the entire infusion packet used to enclose the ingredients be subject to the liquid. With this application there is no need to consider trans-delivery through and/or across a membrane. First of all such delivery dispenses with the mess of the removal of the infusion packet from the liquid. Second the material of the edible film may add active ingredients to the liquid to enhance, sweeten, color. etc. the beverage. Further, it ensures that all the contents will be used. Further, it can be a source of entertainment to, especially children, those who like excitement and magic. U.S. Pat. No 5,089,307 to Niomiya, Suzuki, Ishii (Mitsubishi Rayon Lt. Toyko JP.) title, Edible film and method of making same. If so designed that the soluble film will dissolve in room temperature water_then this would be most desirable to add to the water bottle infusion packet invention.

### Compartments

By providing packets of this nature-infusion packets having ingredients in separate compartments but within the same packet allows for incompatible ingredients when stored but compatible and synergistic when mixed realize their full potential. Ingredients such as pro-biotics and pre-biotics, enzymes, etc. cannot be presented in a ready mixed solution. Including encapsulations of ingredients which do not taste good will allow less sweeteners to be used. Also less chemicals to be used. The inventor hope that this will address the over abundance of sugar necessary to mask flavor. Further this will reduce and/or eliminate the need for food acids present in all ready mixed beverages. While these acids may be used for flavoring their main purpose is to address the microbial growth of organisms within the product. The inventor is most concerned with microbial growths having dealt with the same issue.

Further, it is important to note that extreme attention is given to the active ingredients which are contained within the infusion packet, and or impregnated into the infusion packet. Every effort is made to use existing and new technology to best serve the parameters of each ingredient. To best do so it may warrant the necessity of having more than one compartment. U. S. Pat. No. 5,728,681 to Kido, Kodaira, Munechika, Li, Abe, Yokoyama (The Green Cross Corp. Osaka, JP) titled Infusion preparation and two compartment container containing the preparation. Here is taught the separation of liquids whereby a separator divides the compartments. When removed the liquids in both compartments mix.

While the end result may present a packet with multiple compartments like a quilt it is also conceivable that single units be grouped and connected with a support member.

This may be accomplished in the following ways. One way is to have multiple packets suspended from one central unit be it a string, a straw, a rigid form or a coil resembling a spring (Slinky). These multiple compartments may be grouped all in one area, possibly the end, thus resembling a bunch of cherries. Alternately, they may be staggered along what would resemble a vine and look like flowers on a vine. To this end each packet could be the shape of a flower or bare with it a flower print design on the packet. While in the former, cherry -like formation the individual bags could have thread loops on the end whereby they would slip onto the stalk portion. It is also conceivable that the support member have a clip(s) on the end by which to attach these packets. In the case of a flower stalk/vine little hooks and or clips would be provided going up the vertical. Of special note is that if on chooses to use the latter, the stalk/vine, it would be recommended for a larger vessel like a pitcher for function and beauty. Fig. 11 illustrates a special support member 44 used to combine a number of disparate infusion packets. This allows a mix and match approach to beverages as well as serving as a means of keeping incompatible ingredients separate. Fig 11A shows a "quilt" wherein the support member 44 is used to join individual packets 10. Fig 11 B shows a section through the packets 10 to emphasize the variation in internal contents.

It is conceivable that a boxed unit of infusion packets may contain one or more central units and then an entire selection of infusion packets labeled as to the color, aroma, active ingredients etc. so that the consumer, by choice, may make individual and personal unique combinations.

In addition to the equivalents of the claimed elements, obvious substitutions known to one with ordinary skill in the art are defined to be within the scope of the defined elements. The claims are thus to be understood to include what is specifically illustrated and described above, what is conceptually equivalent, what can be obviously substituted and also what essentially incorporates the essential idea of the invention. The illustrated embodiment has been set forth only for the purposes of example and that should not be taken as limiting the invention. Therefore, it is to be understood that, within the scope of the appended claims, the invention may be practiced other than as specifically described herein.

## Claims

1. An infusion packet (10) for preparing a beverage wherein an aliquot of water-soluble ingredients are surrounded by a water permeable membrane that envelops said ingredients and is sealed so as to form a packet wherein said ingredients cannot penetrate said membrane until said packet is immersed in liquid and a tag (16) that is tethered to said packet (10) **characterized by** having at least two separate compartments containing different ingredients so as to separate incompatible ingredients with at least one of the compartments containing an aliquot of water-soluble dietary fiber and at least one of the compartments containing a different ingredient and wherein the compartments are disposed in a quilted arrangement.

2. The infusion packet (10) of Claim 1, wherein the water soluble dietary fiber is selected from the group consisting of plant mucilage, plant gums, dextrins, maltodextrins, galactomannans, arabanogalactans, beta glucans, cellulose ethers, pectins, pectic material, water-soluble hemicellulose, inulin, alginates, agar, carrageenan, psyllium, guar gum, gum tragarith, gum karya, gum ghatti, gum acacia, gum arabic, partially hydrolyzed products thereof and mixtures thereof.

3. The infusion packet (10) of Claim 1 further comprising ingredients selected from the group consisting of probiotics, prebiotics, flavorings, encapsulated substances, coloring material, amino acids, antioxidants, enzymes, nutraceuticals and dietary supplements.

4. The infusion packet (10) of Claim 1 formulated for managing constipation, management of diabetes, management of obesity, stimulation of the immune system, appetite suppression, lowering serum cholesterol levels, or management of diarrhea.

5. A method for preparing a functional nutraceutical beverage comprising the steps of:
providing an infusion packet (10) of Claim 1; and
immersing said infusion packet in water whereby said fiber and any other ingredients are dissolved and penetrate the membrane to make a functional nutraceutical beverage.

## Patentansprüche

1. Aufgusspäckchen (10) zum Zubereiten eines Getränks, wobei ein Aliquot von wasserlöslichen Inhaltsstoffen von einer wasserdurchlässigen Membrane umgeben ist, die die Inhaltsstoffe umhüllt und versiegelt ist, um ein Päckchen zu bilden, wobei die Inhaltsstoffe nicht durch die Membrane dringen können, bis das Päckchen in Flüssigkeit eingetaucht ist, und ein Anhängeschildchen (16), das an das Päckchen (10) angebunden ist,
**dadurch gekennzeichnet, dass**
es zumindest zwei separate Abteilungen aufweist, die unterschiedliche Inhaltsstoffe enthalten, um unverträgliche Inhaltsstoffe mit zumindest einer der ein Aliquot von wasserlöslicher Nahrungsfaser enthaltenden Abteilungen und zumindest einer der einen anderen Inhaltsstoff enthaltenden Abteilungen zu trennen, und wobei die Abteilungen in einer gesteppten Anordnung eingeteilt sind.

2. Aufgusspäckchen (10) nach Anspruch 1, worin die wasserlösliche Nahrungsfaser ausgewählt ist aus der Gruppe bestehend aus Pflanzenschleim, Pflanzengummen, Dextrinen, Maltodextrinen, Galaktomannanen, Arabanogalaktanen, Betaglukanen, Zelluloseethern, Pektinen, Pektinstoffen, wasserlöslicher Hemizellulose, Inulin, Alginaten, Agar, Carrageenan, Psyllium, Guargummi, Tragantgummi, Karayagummi, Ghattigummi, Akaziengummi, Gummiarabikum, teilweise hydrolysierten Produkten davon und Mischungen davon.

3. Aufgusspäckchen (10) nach Anspruch 1, das ferner Inhaltsstoffe umfasst, die ausgewählt sind aus der Gruppe bestehend aus Probiotika, Präbiotika, Geschmacksstoffen, eingekapselten Substanzen, Farbstoff, Aminosäuren, Antioxydantien, Enzymen, Nutrazeutika und Nahrungsergänzungsmitteln.

4. Aufgusspäckchen (10) nach Anspruch 1, das formuliert ist zum Behandeln von Verstopfung, zur Behandlung von Diabetes, zur Behandlung von Fettleibigkeit, zur Stimulation des Immunsystems, zur Appetitzügelung, zum Senken des Serumcholesterinspiegels oder zur Behandlung von Durchfall.

5. Verfahren zum Zubereiten eines funktionellen nutrazeutischen Getränks, umfassend die Schritte:
- Bereitstellen eines Aufgusspäckchens (10) nach Anspruch 1; und
- Eintauchen des Aufgusspäckchens in Wasser, wodurch die Faser und anderweitige Inhaltsstoffe aufgelöst werden und durch die Membrane dringen, um ein funktionelles nutrazeutisches Getränk abzugeben.

## Revendications

1. Sachet pour infusion (10) pour préparer une boisson, dans lequel une aliquote d'ingrédients solubles dans l'eau est entourée par une membrane perméable à l'eau qui enveloppe lesdits ingrédients et qui est scellée de façon à former un sachet, lesdits ingrédients ne pouvant pas traverser ladite membrane tant que ledit sachet n'est pas plongé dans un liquide, et une étiquette (16) est attachée audit sachet (10), **caractérisé en ce qu'**il possède au moins deux compartiments séparés contenant des ingrédients différents de façon à séparer des ingrédients incompatibles, l'un au moins des compartiments contenant une aliquote de fibre diététique soluble dans l'eau et l'un au moins des compartiments contenant un ingrédient différent, et **en ce que** les compartiments sont disposés selon un agencement piqué.

2. Sachet pour infusion (10) selon la revendication 1, dans lequel la fibre diététique soluble dans l'eau est choisie dans le groupe constitué par un mucilage végétal, des gommes végétales, des dextrines, des maltodextrines, des galactomannanes, des arabinogalactanes, le bêtaglucane, des éthers de cellulose, des pectines, des matières pectiques, l'hémicellulose soluble dans l'eau, l'inuline, des alginates, l'agar-agar, le carraghénane, le psyllium, la gomme de guar, la gomme adragante, la gomme de karaya, la gomme ghatti, la gomme d'acacia, la gomme arabique, ainsi que des produits partiellement hydrolysés et des mélanges de ces substances.

3. Sachet pour infusion (10) selon la revendication 1, comprenant en outre des ingrédients choisis dans le groupe constitué par des probiotiques, des prébiotiques, des arômes, des substances encapsulées, des matières colorantes, des acides aminés, des antioxydants, des enzymes, des nutraceutiques et des compléments diététiques.

4. Sachet pour infusion (10) selon la revendication 1, formulé pour la prise en charge de la constipation, la prise en charge du diabète, la prise en charge de l'obésité, la stimulation du système immunitaire, la suppression de l'appétit, l'abaissement des taux de cholestérol sérique ou la prise en charge de la diarrhée.

5. Procédé de préparation d'une boisson nutraceutique fonctionnelle, comprenant les étapes consistant à :
obtenir un sachet pour infusion (10) selon la revendication 1 ; et
plonger ledit sachet pour infusion dans de l'eau de façon que ladite fibre et les éventuels autres ingrédients se dissolvent et traversent la membrane afin de préparer une boisson nutraceutique fonctionnelle.
